# FASCICULE DE BREVET EUROPEEN

(11) **EP 1 774 959 B1**
(45) Date de publication et mention de la délivrance du brevet: **24.01.2018**
(21) Numéro de dépôt: 06291443.7
(22) Date de dépôt: 13.09.2006
(51) Int. Cl.: A61K 8/60, A61Q 19/02, A61K 31/713, A61K 31/7105, C12N 15/113

(54) **OLIGONUCLÉOTIDE D'ARN DOUBLE BRIN INHIBANT L'EXPRESSION DE LA TYROSINASE**
DOPPELSTRÄNGIGES RNA OLIGONUKLEOTID ZUR HEMMUNG DER EXPRESSION DER TYROSINASE
DOUBLE STRANDED RNA OLIGONUCLEOTIDE FOR THE INHIBITION OF TYROSINASE EXPRESSION

(30) Priorité: 21.09.2005 FR 0509658
(43) Date de publication de la demande: 18.04.2007
(73) Titulaire: L'Oréal, 75008 Paris (FR)
(72) Inventeur: Collin-Djangone, Christine, 60110 Amblainville (FR); Simonnet, Jean-Thierry, 94240 Cachan (FR)
(74) Mandataire: Pillet, Anne-Helene

(56) Documents cités:
- WO-A-2005/042031
- WO-A-2005/060536
- FR-A- 2 840 217
- FR-A- 2 864 540
- FR-A1- 2 804 960
- US-A1- 2004 215 006
- KIM DONG-HO ET AL: "Synthetic dsRNA Dicer substrates enhance RNAi potency and efficacy" NATURE BIOTECHNOLOGY, NATURE PUBLISHING GROUP, NEW YORK, NY, US, vol. 23, no. 2, 26 décembre 2004 (2004-12-26), pages 222-226, XP002399884 ISSN: 1087-0156
- DATABASE WPI Section Ch, Week 200561 Derwent Publications Ltd., London, GB; Class B04, AN 2005-592308 XP002393943 & CN 1 600 858 A (XINYANG BIOTECH CO LTD FUDAN SHANGHAI) 30 mars 2005 (2005-03-30)
- DATABASE WPI Week 200145 Derwent Publications Ltd., London, GB; AN 2001-418809 XP002420964 & CN 1 293 038 A (JIANG N) 2 mai 2001 (2001-05-02)
- KEILHOLZ U ET AL: "Quantitative Detection of Circulating Tumor Cells in Cutaneous and Ocular Melanoma and Quality Assessment by Real-Time Reverse Transcriptase-Polymerase Chain Reaction" CLINICAL CANCER RESEARCH 01 MAR 2004 UNITED STATES, vol. 10, no. 5, 1 mars 2004 (2004-03-01), pages 1605-1612, XP002420945 ISSN: 1078-0432
- DATABASE EBI 2 novembre 2000 (2000-11-02), XP002420681 Database accession no. AAC83357 & WO 00/65067 A2 (UNIV WASHINGTON [US]; NELSON PETER S [US]; HOOD LEROY [US]; LIN BIAOYA) 2 novembre 2000 (2000-11-02)
- DATABASE EBI 5 janvier 2006 (2006-01-05), XP002420682 Database accession no. AEF88668 & WO 2006/002433 A2 (VERIDEX LLC [US]; WANG YIXIN [US]; TALANTOV DIMITRI [US]; MAZUMDER ABH) 5 janvier 2006 (2006-01-05)
- DATABASE EMBL 12 mars 1998 (1998-03-12), XP002420922 Database accession no. A70086 & WO 98/10095 A (BRAX GENOMICS LTD [GB]; SCHMIDT GUNTER [GB]; THOMPSON ANDREW HUGIN [GB) 12 mars 1998 (1998-03-12)
- DATABASE EBI 27 mai 2004 (2004-05-27), XP002420786 Database accession no. ADO48643 & WO 2004/044163 A2 (SEQUENOM INC [US]) 27 mai 2004 (2004-05-27)
- DATABASE EBI 9 février 2006 (2006-02-09), XP002420787 Database accession no. AEF83148 & WO 2006/013610 A (UNIV ROMA [IT]; PASTEUR INSTITUT [FR]; INST NAT SANTE RECH MED [FR]; B) 9 février 2006 (2006-02-09)
- DATABASE EMBL 9 février 2006 (2006-02-09), XP002420923 Database accession no. CS272522 & WO 2006/014477 A1 (BIOREN INC [US]; CREA ROBERTO [US]; RAJPAL ARVIND [US]; TAKEUCHI TOSHI) 9 février 2006 (2006-02-09)
- DATABASE EBI 1 décembre 2005 (2005-12-01), XP002420866 Database accession no. AED87817 & US 2005/266414 A1 (LYONS LESLIE A [US] ET AL) 1 décembre 2005 (2005-12-01)
- DATABASE EBI 28 octobre 1999 (1999-10-28), XP002420867 Database accession no. AAZ38389 & WO 99/54465 A2 (WARNER LAMBERT CO [US]; UNIV IOWA RES FOUND [US]; MIN JING [US]; PESSI) 28 octobre 1999 (1999-10-28)
- DATABASE EBI 12 septembre 2002 (2002-09-12), XP002420868 Database accession no. ABQ82554 & WO 02/070751 A (UNIV PITTSBURGH [US]) 12 septembre 2002 (2002-09-12)
- DATABASE EBI 2 juin 2004 (2004-06-02), XP002420869 Database accession no. AED29973 & CN 1 500 808 A (LI HONG [CN]) 2 juin 2004 (2004-06-02)
- DATABASE EBI 30 juin 2005 (2005-06-30), XP002420870 Database accession no. AEB21524 & WO 2005/059125 A (COMMW SCIENT IND RES ORG [AU]; HORNE IRENE [AU]; KHURANA JEEVAN LAL [A) 30 juin 2005 (2005-06-30)
- DATABASE EBI 7 octobre 2003 (2003-10-07), XP002420680 Database accession no. ADG73679 & JP 2003 284553 A (SEIKAGAKU KOGYO CO LTD) 7 octobre 2003 (2003-10-07)
- DATABASE EBI 23 octobre 2003 (2003-10-23), XP002420939 Database accession no. ADH10948 & WO 03/087331 A (MARICAL INC [US]; HARRIS JR H WILLIAM [US]; NEARING JACQUELINE [US]; B) 23 octobre 2003 (2003-10-23)
- DATABASE EBI 17 août 2005 (2005-08-17), XP002420685 Database accession no. AEC16052 & EP 1 564 286 A (UNIV LIEGE [BE]) 17 août 2005 (2005-08-17)
- DATABASE EBI 16 mai 2002 (2002-05-16), XP002420940 Database accession no. AAL47405 & WO 02/38755 A (JEFFERIES WILFRED A [CA]; CHOI KYUNG BOK [CA]; CHENG NICK [CA]; PAYNE) 16 mai 2002 (2002-05-16)
- DATABASE EBI 15 mai 2003 (2003-05-15), XP002420687 Database accession no. ADJ95343 & WO 03/040325 A2 (CURAGEN CORP [US]; AGEE MICHELE L [US]; ALSOBROOK JOHN P II [US]; BERG) 15 mai 2003 (2003-05-15)
- DATABASE EBI 5 juin 2003 (2003-06-05), XP002420960 Database accession no. ACI10294 & US 2003/104410 A1 (MITTMANN MICHAEL P [US]) 5 juin 2003 (2003-06-05)
- DATABASE EBI 28 mai 2004 (2004-05-28), XP002420879 Database accession no. AED87816 & US 2005/266414 A1 (LYONS LESLIE A [US] ET AL) 1 décembre 2005 (2005-12-01)
- DATABASE EBI 4 juin 1996 (1996-06-04), XP002420943 Database accession no. AAT33319 & JP 08 140699 A (POLA CHEM IND INC) 4 juin 1996 (1996-06-04)
- DATABASE EBI 16 juin 2005 (2005-06-16), XP002420880 Database accession no. AEA42820 & US 2005/130181 A1 (MCSWIGGEN JAMES [US]) 16 juin 2005 (2005-06-16)
- DATABASE EBI 13 avril 2000 (2000-04-13), XP002420885 Database accession no. AAA30125 & WO 00/20460 A (LUDWIG INST CANCER RES [US]; SAHIN UGUR [DE]; TURECI OZLEM [DE]; PFREU) 13 avril 2000 (2000-04-13)
- DATABASE EBI 24 juillet 2003 (2003-07-24), XP002420904 Database accession no. ADC06611 & WO 03/059384 A (US GOVERNMENT [US]; PLETNEV ALEXANDER G [US]; PUTNAK JOSEPH R [US]; CH) 24 juillet 2003 (2003-07-24)
- DATABASE EBI 12 septembre 2002 (2002-09-12), XP002420905 Database accession no. ABQ82554 & WO 02/070751 A (UNIV PITTSBURGH [US]) 12 septembre 2002 (2002-09-12)
- DATABASE EBI 7 juin 2001 (2001-06-07), XP002420906 Database accession no. AAD08997 & WO 01/40475 A2 (UNIV HAWAII [US]) 7 juin 2001 (2001-06-07)
- DATABASE EBI 17 juillet 2003 (2003-07-17), XP002420961 Database accession no. ADA02308 & WO 03/057146 A2 (SAGRES DISCOVERY [US]; MORRIS DAVID W [US] SAGRES DISCOVERY INC [US];) 17 juillet 2003 (2003-07-17)
- DATABASE EMBL 19 septembre 2002 (2002-09-19), XP002420924 Database accession no. AX612813 & WO 02/072882 A2 (OGHAM GMBH [DE]; CULLEN PAUL [DE]; SEEDORF UDO [DE]) 19 septembre 2002 (2002-09-19)
- DATABASE EBI 27 mai 1995 (1995-05-27), XP002420925 Database accession no. AAQ91529 & CA 2 136 705 A1 (CLARKE INST OF PSYCHIATRY [CA]) 27 mai 1995 (1995-05-27)
- DATABASE EBI 15 décembre 2005 (2005-12-15), XP002420962 Database accession no. AEE89228 & WO 2005/118810 A (GENOMICS RES PARTNERS PTY LTD [AU]; BRANDON RICHARD BRUCE [AU]; THOMAS) 15 décembre 2005 (2005-12-15)
- DATABASE EBI 5 juin 2003 (2003-06-05), XP002420926 Database accession no. ACI46460 & US 2003/104410 A1 (MITTMANN MICHAEL P [US]) 5 juin 2003 (2003-06-05)
- DATABASE EBI 6 avril 2000 (2000-04-06), XP002420927 Database accession no. AAA12704 & WO 00/18926 A (UNIV QUEENSLAND [AU]; SOUTHERN CROSS UNIVERSITY [AU]; GRAINS RES & DEV) 6 avril 2000 (2000-04-06)
- DATABASE EBI 30 mai 2002 (2002-05-30), XP002420928 Database accession no. ABS53220 & US 2002/064876 A1 (YOON KYONGGEUN [US]) 30 mai 2002 (2002-05-30)
- DATABASE EBI 1 décembre 2005 (2005-12-01), XP002420929 Database accession no. AEE21703 & US 2005/266410 A1 (WALSH EMILY [US] ET AL) 1 décembre 2005 (2005-12-01)
- DATABASE EBI 21 décembre 1995 (1995-12-21), XP002420930 Database accession no. AAT09327 & WO 95/34680 A (UNIV COLUMBIA [US]; FISHER PAUL B [US]) 21 décembre 1995 (1995-12-21)
- DATABASE EBI 20 mars 2003 (2003-03-20), XP002420931 Database accession no. ACC79427 & WO 03/022307 A (ACAMBIS RES LTD [GB]; TURNER ARTHUR KEITH [GB]; GREENWOOD JUDITH [GB];) 20 mars 2003 (2003-03-20)

### SEQUENCE LISTING

<110> L'Oréal SA
<120> Oligonucléotide d'ARN double brin inhibant l'expression de la tyrosinase
<130> OA05362/CB
<160> 48
<170> PatentIn version 3.3
<210> 1
   <211> 25
   <212> DNA
   <213> Homo sapiens
<400> 1
   gcguaauccu ggaaaccaug acaaa 25
<210> 2
   <211> 25
   <212> DNA
   <213> homo sapiens
<400> 2
   gccuaccuca cuuuagcaaa gcaua 25
<210> 3
   <211> 25
   <212> DNA
   <213> homo sapiens
<400> 3
   ccugugucuc cucuaagaac cugau 25
<210> 4
   <211> 25
   <212> DNA
   <213> Homo sapiens
<400> 4
   ccauagggac cuauggccaa augaa 25
<210> 5
   <211> 25
   <212> DNA
   <213> Homo sapiens
<400> 5
   ccgauuggag gaguacaaca gccau 25
<210> 6
   <211> 25
   <212> DNA
   <213> Homo sapiens
<400> 6
   ggaccuuuac ggcguaaucc uggaa 25
<210> 7
   <211> 25
   <212> DNA
   <213> Homo sapiens
<400> 7
   ggcguaaucc uggaaaccau gacaa 25
<210> 8
   <211> 25
   <212> DNA
   <213> Homo sapiens
<400> 8
   ccugucagaa uauccuucug uccaa 25
<210> 9
   <211> 25
   <212> DNA
   <213> Homo sapiens
<400> 9
   ccuggaaacc augacaaauc cagaa 25
<210> 10
   <211> 25
   <212> DNA
   <213> Homo sapiens
<400> 10
   ccaauaugaa ucugguucca uggau 25
<210> 11
   <211> 25
   <212> DNA
   <213> homo sapiens
<400> 11
   gguuccaugg auaaagcugc caauu 25
<210> 12
   <211> 25
   <212> DNA
   <213> Homo sapiens
<400> 12
   gcaaagcaua ccaucagcuc agacu 25
<210> 13
   <211> 25
   <212> DNA
   <213> Homo sapiens
<400> 13
   ggaguacaac agccaucagu cuuua 25
<210> 14
   <211> 25
   <212> DNA
   <213> Homo sapiens
<400> 14
   gcagagguuc cugucagaau auccu 25
<210> 15
   <211> 25
   <212> DNA
   <213> Homo sapiens
<400> 15
   gcagauuguc uguagccgau uggag 25
<210> 16
   <211> 25
   <212> DNA
   <213> Homo sapiens
<400> 16
   gaucaacacc cauguuuaac gacau 25
<210> 17
   <211> 25
   <212> DNA
   <213> Homo sapiens
<400> 17
   gaguacaaca gccaucaguc uuuau 25
<210> 18
   <211> 25
   <212> DNA
   <213> Homo sapiens
<400> 18
   gcauuauuau gugucaaugg augca 25
<210> 19
   <211> 25
   <212> DNA
   <213> Homo sapiens
<400> 19
   gaccuuuacg gcguaauccu ggaaa 25
<210> 20
   <211> 25
   <212> DNA
   <213> Homo sapiens
<400> 20
   gccuuggcau agacucuucu uguug 25
<210> 21
   <211> 25
   <212> DNA
   <213> Homo sapiens
<400> 21
   gcacagauga guacauggga gguca 25
<210> 22
   <211> 25
   <212> DNA
   <213> Homo sapiens
<400> 22
   gagagacgac ucuuggugag aagaa 25
<210> 23
   <211> 25
   <212> DNA
   <213> Homo sapiens
<400> 23
   gaggaguaca acagccauca gucuu 25
<210> 24
   <211> 25
   <212> DNA
   <213> Homo sapiens
<400> 24
   ucuguagccg auuggaggag uacaa 25
<210> 25
   <211> 25
   <212> DNA
   <213> Homo sapiens
<400> 25
   ucccuagagc cugugucucc ucuaa 25
<210> 26
   <211> 25
   <212> DNA
   <213> Homo sapiens
<400> 26
   ucuuggcaga uugucuguag ccgau 25
<210> 27
   <211> 25
   <212> DNA
   <213> Homo sapiens
<400> 27
   ucuuguugcg gugggaacaa gaaau 25
<210> 28
   <211> 25
   <212> DNA
   <213> Homo sapiens
<400> 28
   gccaucaguc uuuaugcaau ggaac 25
<210> 29
   <211> 25
   <212> DNA
   <213> Homo sapiens
<400> 29
   ccaaacugca cagagagacg acucu 25
<210> 30
   <211> 25
   <212> DNA
   <213> Homo sapiens
<400> 30
   ccugaguuug acccaauaug aaucu 25
<210> 31
   <211> 25
   <212> DNA
   <213> Homo sapiens
<400> 31
   ccaauuagcc aguuccugca gaccu 25
<210> 32
   <211> 25
   <212> DNA
   <213> Homo sapiens
<400> 32
   ggaggaguac aacagccauc agucu 25
<210> 33
   <211> 25
   <212> DNA
   <213> Homo sapiens
<400> 33
   ccauggauaa agcugccaau uucag 25
<210> 34
   <211> 25
   <212> DNA
   <213> Homo sapiens
<400> 34
   acucuucuug uugcgguggg aacaa 25
<210> 35
   <211> 25
   <212> DNA
   <213> Homo sapiens
<400> 35
   acaacagcca ucagucuuua ugcaa 25
<210> 36
   <211> 25
   <212> DNA
   <213> Homo sapiens
<400> 36
   accucacuuu agcaaagcau accau 25
<210> 37
   <211> 25
   <212> DNA
   <213> Homo sapiens
<400> 37
   gucuggaugc auuauuaugu gucaa 25
<210> 38
   <211> 25
   <212> DNA
   <213> Homo sapiens
<400> 38
   accuugugag gacuagagga agaau 25
<210> 39
   <211> 25
   <212> DNA
   <213> Homo sapiens
<400> 39
   guuccaugga uaaagcugcc aauuu 25
<210> 40
   <211> 25
   <212> DNA
   <213> Homo sapiens
<400> 40
   acagagagac gacucuuggu gagaa 25
<210> 41
   <211> 25
   <212> DNA
   <213> Homo sapiens
<400> 41
   gccugugucu ccucuaagaa ccuga 25
<210> 42
   <211> 25
   <212> DNA
   <213> Homo sapiens
<400> 42
   ucaggcagag guuccuguca gaaua 25
<210> 43
   <211> 25
   <212> DNA
   <213> Homo sapiens
<400> 43
   caacagccau cagucuuuau gcaau 25
<210> 44
   <211> 25
   <212> DNA
   <213> Homo sapiens
<400> 44
   gcauaccauc agcucagacu auguc 25
<210> 45
   <211> 25
   <212> DNA
   <213> homo sapiens
<400> 45
   ccaucagucu uuaugcaaug gaacg 25
<210> 46
   <211> 25
   <212> DNA
   <213> homo sapiens
<400> 46
   ggaucaacac ccauguuuaa cgaca 25
<210> 47
   <211> 25
   <212> DNA
   <213> homo sapiens
<400> 47
   cagagguucc ugucagaaua uccuu 25
<210> 48
   <211> 19
   <212> DNA
   <213> homo sapiens
<400> 48
   ugcaccacuu gggccucaa 19

## Description

La présente invention se rapporte à de nouveaux oligonucléotides d'ARN double brin permettant de diminuer l'expression de la tyrosinase, à leur utilisation à des fins cosmétiques et/ou pharmaceutiques ainsi qu'à leur association avec des particules cationiques d'une taille inférieure ou égale à 1 µm, de potentiel zeta compris entre 10 et 80 mV.

La tyrosinase (monophénol dihydroxyl phénylalanine : oxygen oxydo-reductase EC 1.14.18.1) est l'enzyme essentielle intervenant dans le mécanisme de pigmentation de la peau. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. La tyrosinase n'agit que lorsqu'elle est à l'état de maturation sous l'action de certains facteurs biologiques.

Cette enzyme peut également trouver un intérêt dans le traitement de pathologies telles que le mélanome (Riley et al., J. Immunother. 2001, 21, 212-220) ou la maladie de Vogt-Koyanagi-Harada (Read et al., Curr. Opin. Ophthalmol., 2000, 11, 437-442).

Le mécanisme de formation de la pigmentation de la peau, c'est-à-dire de la formation de la mélanine est particulièrement complexe et fait intervenir schématiquement les principales étapes suivantes :
**Tyrosine → Dopa → Dopaquinone → Dopachrome → Mélanine**

Ainsi, la pigmentation de la peau humaine résulte de la synthèse de mélanine par les cellules dendritiques, les mélanocytes. Ces derniers contiennent des organelles appelées mélanosomes, siège de la biosynthèse de mélanine. Ce sont les mélanosomes qui, après migration le long des dendrites, sont transférés des mélanocytes vers les kératinocytes. Les kératinocytes sont alors transportés à la surface de la peau au cours du processus de la différentiation de l'épiderme (Gilchrest BA, Park HY, Eller MS, Yaar M, Mechanisms of ultraviolet light-induced pigmentation. Photochem Photobiol 1996; 63 : 1-10*;* Hearing VJ, Tsukamoto K, Enzymatic control of pigmentation in mammals. FASEB J 1991; 5 : 2902-2909).
Parmi les enzymes de la mélanogenèse, la tyrosinase est une enzyme clé qui catalyse les deux premières étapes de la synthèse de mélanine. Des mutations homozygotes de la tyrosinase provoquent un albinisme oculocutané de type I caractérisé par une totale absence de synthèse de mélanine (Toyofuku K, Wada I, Spritz RA, Hearing VJ, The molecular basis of oculocutaneous albinism type 1 (OCA1): sorting failure and degradation of mutant tyrosinases results in a lack of pigmentation. Biochem J. 2001; 355: 259-269).

Les désordres de l'hyperpigmentation résultant d'un accroissement de la production de mélanine, de nouvelles approches thérapeutiques dont le rationnel est basé sur l'inhibition de l'activité de la tyrosinase s'avèrent importantes à développer.
La plupart des composés éclaircissants de la peau déjà connus sont des phénols/catéchols. Ces composés inhibent la tyrosinase mais la majorité de ces composés sont cytotoxiques envers les mélanocytes, ce qui pourrait provoquer une dépigmentation permanente de la peau.

Il apparaît donc intéressant, pour une application chez l'homme, de disposer de nouveaux composés inhibiteurs de la tyrosinase ayant à la fois une bonne efficacité et une bonne tolérance.

Depuis peu, l'utilisation d'oligonucléotides d'ARN double brin, dsRNA et plus particulièrement de siRNA (de 12 à 40 nucléotides), permettrait d'obtenir une activité spécifique dans le domaine cosmétique tel que le soin de la peau ou le soin capillaire, mais aussi dans les domaines dermatologique et pharmaceutique.

Cependant, l'utilisation *in vivo* des siRNA est connue pour présenter différentes difficultés. Outre le problème de la pénétration de ces siRNA pour atteindre les cellules cibles lors de leur application topique en raison notamment de la difficulté à traverser le *stratum corneum,* l'expérience a montré que l'administration de siRNA pouvait conduire au déclenchement d'une réponse interféron rapporté par de nombreuses publications (Sledz CA et al., Activation of the interferon system by short-interfering RNAs. Nat Cell Biol. 2003 ;9:834-9*.* Katalin Karikó et al., Small Interfering RNAs Mediate Sequence-Independent Gene Suppression and Induce Immune Activation by Signaling through Toll-Like Receptor 31. J. Immunol. 2004; 172: 6545-6549*.* Judge AD et al. Sequence-dependent stimulation of the mammalian innate immune response by synthetic siRNA. Nat Biotechnol. 2005 ;23:457-62) ainsi qu'à l'induction ou à l'inhibition de l'expression de gènes non ciblés par le siRNA (Jackson et al. Expression profiling reveals off-target gene regulation by RNAi. Nat Biotechnol. 2003;21:635-7), ces deux phénomènes sont hautement indésirables.

La demande de brevet US 2004/0215006 décrit des oligonucléotides d'ARN simple brin anti-sens et double brin actifs contre la tyrosinase, les exemples mis en oeuvre se rapportent uniquement à des oligonucléotides simple brin d'ARN anti-sens. En particulier, il n'est pas démontré que les siRNA homologues à ces ARN anti-sens sont efficaces.
Certains auteurs ont souligné que les oligonucléotides d'ARN double brin, tels que les siRNA, et les oligonucléotides simple brin d'ARN antisens avaient des cibles différentes et que l'activité d'un ARN antisens n'était pas extrapolable au siRNA de même séquence (Xu et al., Effective small interfering RNAs and phosphorothioate antisense DNAs have different preferences for target sites in the luciferase mRNAs. BBRC 2003;306 :712-717)*.*
Par ailleurs, dans ce document US 2004/0215006, les concentrations en siRNA proposées se situent entre 50 et 200 nM. Comme indiqué plus haut, il a été décrit par Jackson *et al.* une forte régulation positive et négative de gènes non ciblés par les siRNA utilisés à des concentrations de 100 nM. De la même façon, dans le WO 2005/060536 qui décrit des siRNA inhibant spécifiquement la tyrosinase, les gammes de concentration proposées sont très large et peuvent conduire à des compositions comprenant une très grande quantité de siRNA pour lesquelles on pourra constater une régulation positive et négative de gènes non ciblés par les siRNA. Dans le cadre d'une utilisation cosmétique ou thérapeutique, cette régulation de gènes non ciblés n'est pas acceptable, notamment en raison de son caractère imprévisible.
FR2 840 217 décrit l'utilisation cosmétique d'oligonucléotides double brin (dsRNA ou siRNA). Ce document divulgue différentes cibles dont l'expression peut être inhibée par des dsRNA à des fins cosmétiques, notamment la tyrosinase, et propose plusieurs séquences spécifiques d'oligonucléotides. WO 2005/042031 décrit des oligonucléotides doubles brins capables de décomposer des ARNm de protéines impliquées dans la pigmentation de la peau et des cheveux. Par ailleurs, l'article de Kim Dong-Ho et al. (« Synthetic dsRNA Dicer substrates enhance RNAi potency and efficacy », Nature Biotechnology, Vol. 23, N°2, 2004, pages 222-226*)* étudie l'effet de la longueur des siRNA sur leur activité.

La demanderesse a mis au point de nouveaux siRNA inhibant spécifiquement l'expression de la tyrosinase parmi lesquels elle a sélectionné des siRNA présentant une activité telle que ces siRNA peuvent être utilisés à une dose inférieure ou égale à 1 nM.
En plus de résoudre les problèmes liés à l'induction de gènes non spécifiques, la demanderesse a vérifié que les siRNA avec ces séquences n'induisaient pas de réponse interferon.
Pour cela, elle a mesuré l'expression des gènes OAS-1 et IFIT-1 (interferon-inducible tetratricopeptide repeat domain) connus pour être induits par l'interféron (Stefan F. Wieland et al., Searching for Interferon-Induced Genes That Inhibit Hepatitis B Virus Replication in Transgenic Mouse Hepatocytes, J. of Virology 2003,77 :1227-1236) selon le protocole décrit dans le manuel du kit « BLOCK-iT™ RNAi Stress Response Control Kit (Human) For monitoring interferon-mediated stress response to double-stranded RNA in human cells » commercialisé par Invitrogen.

En outre, l'association de ces siRNA spécifiques à la tyrosinase avec des particules cationiques d'une taille inférieure ou égale à 1 µm, de potentiel zeta compris entre 10 et 80 mV permet d'améliorer très significativement leur pénétration dans les cellules cibles d'un modèle tridimensionnel tel que la peau. Une fois pénétré, le siRNA spécifique à la tyrosinase devient actif. La pénétration peut être évaluée à l'aide de marqueur fluorescent fixé sur le siRNA et son activité par la quantification du messager ciblé par PCR quantitative ou par le dosage de la protéine correspondante au messager ciblé.
Les particules cationiques de l'invention, peuvent être des micelles de tensioactifs, des micelles de polymères blocs ou non, des liposomes et niosomes cationiques, des oléosomes cationiques, des nanoémulsions cationiques, ainsi que des particules organiques ou inorganiques cationiques et les nanocapsules.

Ainsi, la présente demande décrit un oligonuciéotide d'ARN double brin (aussi appelé dsRNA ou siRNA) choisi parmi les oligonucléotides de séquence SEQ. ID. n°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 et 48, éventuellement modifié.

Un premier objet de l'invention se rapporte à un oligonucléotide d'ARN double brin (aussi appelé dsRNA ou siRNA) choisi parmi les oligonucléotides de séquence SEQ. ID. n°1, 2, 4, 5, 7, 8, 13, 14, 16, 23, 25, 29, 32, 35, 37, 40, 42, 43, 46 ou 47, éventuellement modifié.

L'utilisation de dsRNA et, plus particulièrement, de siRNA (pour «short interfering» RNA) permet d'obtenir une activité d'inhibition spécifique de la synthèse d'une protéine cible par dégradation de l'ARNm codant la protéine. La dégradation de l'ARNm cible est obtenue par l'activation du complexe RISC (RNA Induced Silencing Complex) effective par la fixation du brin anti-sens du dsRNA sur l'ARNm (voir Tuschl T. Chem. Biochem. 2001;2:239-245*;* Nykanen A & al, Cell 2001;107:309-321*;* Dorsett Y. and Tuschl T. Nat Rev Drug Discov. 2004;3:318-329*;* Downward J. BMJ. 2004;328:1245-1248*;* Shankar P. et al., JAMA 2005 ;293:1367-1373)*.*
Le mécanisme moléculaire mis en jeu fait intervenir des fragments d'ARN double brin constitués de 12 à 40 nucléotides, préférentiellement de 23 à 27 nucléotides.
Ces oligonucléotides d'ARN double brin peuvent préférentiellement être composés d'un brin sens homologue et d'un brin anti-sens complémentaire de la séquence du mRNA de la tyrosinase humaine (GenBank accession number NM_000372). L'oligonucléotide d'ARN double brin présente des extrémités franches ou non appariées de 2 à 6 nucléotides.
Les oligonucléotides d'ARN double brin peuvent être synthétisés selon de nombreuses méthodes de synthèse *in vivo* ou *in vitro* de façon manuelle ou automatique.

Les méthodes de synthèse *in vitro* peuvent être chimiques ou enzymatiques, par exemple en utilisant une ARN polymérase (à titre d'exemple de T3, T7 ou SP6) qui réalisera la transcription d'un modèle de séquence d'ADN (ou d'ADNc) choisi.
De nombreuses méthodes de synthèse *in vivo* d'ARN double brin sont décrites dans la littérature, elles peuvent être réalisées dans divers types cellulaires de bactéries ou d'organismes supérieurs (Sambrook et al. Molecular Cloning, A Laboratory Manual, Second Edition (1989*)*, DNA cloning, volume I and II, D. N. Glover (ed. 1985*),* Oligonucleotide Synthesis, M. J. Gaits (ed. 1984*)*, Nucleic Acid Hybridation, B. D. Hames and S. J. Higgins (ed.1984*),* Transcription and Translation B. D. Hames and S. J. Higgins (ed.1984*),* Animal Cell Culture, R. I. Freshney (ed. 1986*),* Immobilised Cells and Enzymes, IRL Press (1986*),* B. Pertal, A Practical Guide to Molecular Cloning (1984*),* Gene Transfer Vectors for Mammalian Cells, J. H. Miller and M. P. Calos, Cold Spring Harbor Laboratory (ed. 1987*),* Methods in Enzymology, vol. 154, Wu and Grossman*, and* 155, Wu, Mayer and Walker (1987*),* Immunochemical Methods in Cell and Molecular Biology, Academic Press, London, Scopes (1987*),* Protein Purification: Principle and Practice, 2nd ed., Springer-Verlag, N.-Y*. and* Handbook of Expérimental Immunology, vol.I-IV, C. D. Weir and C. C. Blackwell (1986*))*. On pourra également se référer aux méthodes de synthèses décrites dans les demandes de brevet WO01/36646, WO01/75164 et US20030088087.
De façon préférentielle, les oligonucléotides d'ARN double brin selon l'invention sont modifiés par l'addition en 2' d'un groupement -O-Methyl.
Les oligonucléotides d'ARN double brin peuvent subir différentes modifications, ils peuvent en particulier être modifiés comme décrit dans le document US 2004014956.
De façon avantageuses, les modifications conduiront à des oligonucléotides d'ARN double brin plus stables et, encore préférentiellement, des oligonucléotides d'ARN double brin rendus furtifs vis-à-vis de la réponse interféron, cette propriété est capitale pour une utilisation *in vivo.* Les oligonucléotides d'ARN double brin pourront encore avoir une séquence sens modifiée de façon qu'elle ne peut être incorporée dans le RISC et donc ne peut induire des effets secondaires.

De façon préférentielle, on utilisera les oligonucléotides d'ARN double brin de SEQ. ID. n°1, 2, 4, 13, 16, 35, 37, 40 et 42 ayant une efficacité de dégradation de l'ARNm de la tyrosinase supérieure à 50% à 0.016 nM mesurée selon le protocole de l'exemple 2 ci-après.

La présente invention décrit également une composition comprenant au moins un oligonucléotide d'ARN double brin choisi parmi les oligonucléotides de séquence SEQ ID. n°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 et 48.

L'objet de la présente invention se rapporte également à une composition comprenant au moins un oligonucléotide d'ARN double brin choisi parmi les oligonucléotides de séquence SEQ. ID. n°1, 2, 4, 5, 7, 8, 13, 14, 16, 23, 25, 29, 32, 35, 37, 40, 42, 43, 46 ou 47.

Dans cette composition, l'oligonucléotide d'ARN double brin est présent à une concentration inférieure ou égale à 100 µM, de préférence, inférieure ou égale à 10 µM et encore préférentiellement à 1 µM.
La composition selon l'invention est de préférence adaptée à une administration topique sur la surface du corps, c'est-à-dire, la peau, les phanères, les muqueuses et les yeux.
Selon la présente invention, les oligonucléotides d'ARN double brin sont associés à une particule cationique d'une taille inférieure ou égale à 1 µm, de potentiel zeta compris entre 10 et 80 mV.
Dans ce mode de réalisation, les oligonucléotides d'ARN double brin vont adhérer à la surface de la particule, celle-ci servant de véhicule pour le faire pénétrer dans les structures cutanées et dans les cellules cibles de la peau, des muqueuses ou encore des yeux pour des applications cosmétiques, dermatologiques et/ou ophtalmiques.
Les particules cationiques selon l'invention sont des particules ayant une taille inférieure ou égalé à 1 µm, de préférence inférieure ou égale à 500 nm, encore préférentiellement inférieure ou égale à 300 nm, mesurable avec, par exemple, un granulométre laser type BI90 plus de la société Brookhaven, et ayant un potentiel zeta compris entre 10 et 80 mV pouvant être mesuré avec un zetamètre type DELSA 440 de la société Coultronics.
La particule cationique peut être choisie parmi les micelles de tensioactifs, en particulier, les micelles de tensioactifs amphiphiles non ioniques et de tensioactifs cationiques, les micelles de polymères blocs, en particulier, les micelles de polymère bloc amphiphile cationique, les micelles de polymère bloc amphiphile non ionique et de polymère bloc amphiphile cationique et les micelles de polymère bloc amphiphile non ionique et de tensioactif cationique, les liposomes de tensioactifs non ionique et cationiques, les niosomes, les oléosomes, les particules de nanoémulsions, les nanocapsules, les particules organiques ou les particules inorganiques.
Sont listées ci-après et de façon non exhaustive des particules cationiques utilisables selon l'invention.

### Micelles de tensioactifs

Pour rappel, les micelles sont les agrégats que forment spontanément les molécules amphiphiles lorsqu'elles sont solubilisées dans l'eau ou l'huile, au-delà d'une certaine concentration dite critique : la CMC.

Les micelles utilisables dans le cadre de l'invention sont constituées d'au moins un tensioactif cationique, Ce tensioactif cationique peut être associé à un ou plusieurs tensioactifs amphiphiles non ioniques.

L'homme du métier sélectionnera avantageusement les tensioactifs non ioniques et cationiques dans le McCutcheons 1998 « emulsifiers and detergents » ainsi que dans les éditions suivantes.

A titre d'exemples non limitatifs, les tensioactifs cationiques utilisables dans le cadre de l'invention sont listés ci-après.

Sans que ce soit limitatif, les tensioactifs non ioniques utilisables sont : les alkyls et polyalkyls (C6 à C30, saturé ou non, ramifié ou non) ester ou ether de POE, de glycérol et de polyglycérol, de sorbitan oxyéthyléné ou non, de sucrose, de glucose oxyethyléné ou non, de maltose, de POP-POE.

En cas de mélange entre tensioactifs non ioniques et tensioactifs cationiques, leurs proportions respectives, en poids, seront comprises entre 99/1 et 1/99.

Le taux de tensioactifs formant les micelles sera dépendant de la CMC de ceux-ci. Cependant, dans le cadre de l'invention, la concentration en tensioactifs micellaires sera comprise entre 0.1 et 10% et de préférence entre 0.2 et 5% en poids par rapport au poids total de la composition.

### Micelles de polymère bloc

Les micelles de polymères blocs amphiphiles peuvent être préparées selon le procédé décrit dans le document WO 04/035013.

Les copolymères blocs utiles pour la préparation des micelles associés aux dsRNA selon l'invention sont notamment des polymères blocs amphiphiles, préférentiellement non ioniques, diblocs ou triblocs, qui peuvent former au contact de l'eau des micelles. Ils sont notamment de type dibloc (A-B) ou tribloc (A-B-A), A correspondant à un bloc polymérique hydrophile non ionique et B à un bloc polymérique hydrophobe. Le poids moléculaire des polymères peut être compris entre 1000 et 100.000 et le rapport A/B peut être compris entre 1/100 et 50/1.
Dans le cadre de l'invention, trois types de micelles peuvent être utilisés :
- des micelles de polymère bloc amphiphile cationique ;
- des micelles mixtes de polymère bloc amphiphile non ionique associé à un polymère bloc amphiphile cationique ; et
- des micelles mixtes de polymère bloc amphiphile non ionique associé à un tensio actif cationique.

Le bloc polymérique hydrophile non ionique peut être choisi parmi le polyoxyde d'éthylène (POE) et la polyvinylpyrrolidone (PVP).

Le bloc polymérique hydrophobe peut être choisi parmi le polystyrène, le poly(tert.-butylstyrène), le poly(méthylméthacrylate), le poly(éthylacrylate), le poly(butylacrylate), le poly(butylméthacrylate), le poly(vinylacétate), les polycaprolactones, les polycaprolactames, les polydiméthylsiloxanes, les polyoxydes d'alkylène en C3-C6, le poly(acide aspartique), le poly(acide lactique), le poly(acide glycolique), la polyleucine, les polybutadiènes, les polyéthylènes, les polypropylènes, les polybutylènes.
Le copolymère bloc est choisi de préférence parmi les copolymères blocs suivants :
- polyoxyde de propylène/polyoxyde d'ethylène
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

Dans le cadre de l'invention, il est nécessaire d'ajouter à la composition micellaire :
- un polymère bloc amphiphile cationique dont l'un des blocs est cationique et peut être choisi parmi, à titre d'exemple :
   - Poly methacrylate de trimethyl ethyl ammonium ;
   - Polymethacrylate de dimethyl amino ethyl quaternisé ;
   - Poly methyl vinyl imidazolium ;
   - Poly vinyl benzyl trimethylamonium chlorure.
   l'association d'un polymère bloc amphiphile non ionique avec un polymère blocs amphiphile cationique est telle que le rapport entre les deux sera compris entre 99/1 et 1/99 ;
   et/ou
- au moins un tensioactif cationique tel que listé ci-après.
Dans ce cas, le rapport respectif entre le polymère bloc amphiphile non ionique et le tensioactif cationique sera compris entre 50/50 et 99/1.

Dans le cadre de l'invention, la concentration en polymères blocs micellaires associés ou non à un tensioactif cationique, sera comprise entre 0,1 et 10% et, de préférence, entre 0,2 et 5% en poids par rapport au poids total de la composition.

Dans une variante de l'invention, on peut également former des micelles constituées de polymères blocs amphiphile cationique tels que décrits ci-dessus.

### Liposomes et niosomes

Les lipides amphiphiles non ioniques aptes à former des liposomes non ioniques sont en particulier ceux décrits dans la demande de brevet EP 0 582 503.

En particulier, les lipides amphiphiles non-ioniques peuvent être constitués par un mélange d'esters d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités oxyde d'éthylène, et d'au moins un acide gras comportant une chaîne alkyle en C5-C17, saturée ou non saturée, linéaire ou ramifiée, le nombre de chaînes alkyle par groupe polyol étant compris entre 1 et 10 .

L'expression "mélange d'esters" couvre non seulement des mélanges d'esters purs de familles chimiques différentes mais couvre également tout produit, qui contient plusieurs esters de polyol chimiquement purs de la même famille dans des proportions variables. C'est, en particulier, le cas des produits ayant une formule statistique, dans leur partie hydrophile, par exemple un ester de polyglycérol de formule CO-(OCH₂-CHOH-CH₂)ₙ-OH où n est une valeur statistique et qui peut contenir des proportions diverses d'esters pour lesquels n = 1, n = 2, n = 3, n = 4, etc ... ; c'est aussi le cas des esters comportant plusieurs chaînes alkyles dans leur partie lipophile, tels que les cocoates, qui contiennent des chaînes alkyles en C5 à C17 ou les isostéarates, où les chaînes alkyles en C17 sont un mélange complexe de formes isomères ; c'est également le cas des produits constitués par des mélanges de mono-, di-, tri-ou polyesters d'un même polyol. Il faut noter qu'un produit, qui ne contiendrait qu'un seul ester susceptible de former des vésicules et des impuretés d'un autre type ne pourrait pas être utilisé selon l'invention.

Des esters commerciaux utilisables seuls selon l'invention, car ils sont en réalité des mélanges d'esters, sont par exemple les suivants :
- les esters partiels de sorbitane (ou anhydride de sorbitol) et d'acide gras, vendus sous les dénominations commerciales "SPAN 20, 40, 60 et 80" par la société "ICI" ;
- l'isostéarate de sorbitane, vendu sous la dénomination commerciale "SI 10 R NIKKOL" par la société "NIKKO" ;
- le stéarate de sorbitane portant 4 unités oxyde d'éthylène, vendu sous la dénomination "TWEEN 61" par la société "ICI" ;
- le stéarate de polyéthylène glycol à 8 unités oxyde d'éthylène, vendu sous la dénomination "MYR J 45" par la société "ICI" ;
- le monostéarate du polyéthylène glycol de formule EMI6.1 formule dans laquelle n est égal à 4, vendu sous la dénomination "MYS 4" par la société "NIKKO" ;
- le stéarate de polyéthylène glycol de poids moléculaire 400, qualité chimique ou qualité produite par biotechnologie, vendu par la société "UNICHEMA" ;
- le stéarate de diglycéryle portant 4 unités d'oxyde d'éthylène, vendu sous la dénomination "HOSTACERINE DGS" par la société "HOECHST" ;
- le stéarate de tétraglycérol, vendu sous la dénomination "TETRAGLYN 1S" par la société "NIKKO" ;
- l'isostéarate de diglycéryle, vendu par la société "SOLVAY" ;
- le distéarate de diglycéryle, vendu sous la dénomination "EMALEX DSG 2" par la société "NIHON" ;
- les mono-, di- et tripalmitostéarate de sucrose, vendus sous les dénominations " F50, F70, F110 ET F160 CRODESTA" par la société "CRODA" ;
   le mélange de mono- et dipalmitostéarate de sucrose, vendu sous la dénomination "GRILLOTEN PSE 141 G" par la société "GRILLO" ;
- le mélange de stéarate de sucrose et de cocoate de sucrose, vendu sous la dénomination "ARLATONE 2121" par la société "ICI" ;
- le distéarate de méthylglucose portant 20 unités oxyde d'éthylène, vendu sous la dénomination "GLUCAM E 20 DISTEARATE" par la société "AMERCHOL".

Des mélanges entre eux de ces différents produits, qui sont déjà des mélanges, ou des mélanges de ces produits avec des produits purs peuvent, bien entendu, être utilisés.

Les tensioactifs cationiques sont choisis dans la liste ci-après et de telle sorte qu'ils confèrent à la dispersion un pH compris entre 5 et 8 ; le rapport en poids entre la quantité de lipides amphiphiles non ioniques et celle de tensioactifs cationiques dans la phase lipidique étant compris entre 50/1 et 50/25 et le rapport en poids entre la phase lipidique et la phase aqueuse de dispersion étant compris entre 1/1 000 et 300/1 000.

### Oléosomes

Les oléosomes relatifs à l'invention sont décrits dans la demande de brevet EP 0 705 593. Il s'agit d'une émulsion du type huile dans eau formée de globules huileux pourvus d'un enrobage cristal liquide lamellaire dispersés dans une phase aqueuse, caractérisée en ce que chaque globule huileux est unitairement enrobé d'une couche monolamellaire ou oligolamellaire (1 à 10 feuillets visualisables en microscopie électronique à transmission après cryofracture) obtenue à partir d'au moins un agent tensioactif lipophile, d'au moins un agent tensioactif hydrophile et d'au moins un tensioactif cationique conférant à l'émulsion un pH allant de 5 à 8, les globules huileux enrobés ayant un diamètre moyen inférieur à 500 nanomètres.

L'agent tensioactif lipophile et l'agent tensioactif hydrophile comportent chacun au moins une chaîne grasse saturée ayant plus de 12 atomes de carbone environ. Plus préférentiellement encore, cette chaîne grasse contient de 16 à 22 atomes de carbone.

Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.

Des exemples de tels agents tensioactifs lipophiles sont le distéarate de sucrose, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythritol.

L'agent tensioactif hydrophile présente de préférence un HLB compris entre environ 8 et environ 12.

On peut citer comme exemples de tels tensioactifs hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthylèné 12 OE et le distéarate de méthylglucose polyoxyéthyléné 20 OE.

Les tensioactifs cationiques peuvent être choisis parmi les composés cités ci-après.

### Nanoémulsions

Les particules cationiques peuvent aussi être choisies parmi les nanoémulsions huile-dans-eau comportant une phase huileuse dispersée dans une phase aqueuse, dont les globules d'huile ont une taille moyenne en nombre inférieure à 100 nm, caractérisées en ce qu'elles comprennent au moins un lipide amphiphile comprenant au moins un lipide amphiphile non ionique et un lipide amphiphile cationique, la phase huileuse et les lipides amhiphiles étant présents en une teneur telle que le rapport pondéral phase huileuse/lipide amphiphile va de 3 à 10.
Les nanoémulsions ont généralement un aspect transparent à bleuté. Leur transparence se mesure par un coefficient de transmittance à 600 nm allant de 10 à 90 % ou bien par une turbidité. La turbidité des compositions de l'invention va de 60 à 400 NTU et de préférence de 70 à 300 NTU, turbidité mesurée au turbidimètre portatif HACH - Modèle 2100 P à environ 25°C.

Les globules d'huile des nanoémulsions de l'invention ont une taille moyenne en nombre, inférieure à 100 nm et de préférence allant de 20 à 80 nm et plus préférentiellement de 40 à 60 nm. La diminution de la taille des globules permet de favoriser la pénétration des actifs dans les couches superficielles de la peau (effet véhicule).

Les nanoémulsions conformes à l'invention sont préparées de préférence à des températures allant de 4 à 45°C et sont ainsi compatibles avec des actifs thermosensibles.

Ces dispersions sont notamment décrites dans les demandes suivantes : EP 0728 460, EP 0 879 589, EP 1 010 413, EP 1 010 414, EP 1 010 416, EP 1 013 338, EP 1 016 453, EP 1 018 363, EP 1 025 898 et EP 1 120 102. Dans toutes ces demandes, il est précisé que pour améliorer la stabilité des particules, on ajoutera un tensioactif ionique au tensioactif non ionique (ou mélange).

Dans le cas de la présente demande, on utilisera comme tensioactif ionique exclusivement un ou des tensioactifs cationiques.
Les proportions indiquées dans les références ci-dessus sont à conserver et à titre d'exemple de tensioactif cationique, on retiendra la liste commune à toutes les particules.

Le **tensioactif non ioniques,** de préférence hydrosolubles ou hydrodispersibles, comportent au moins une séquence hydrophobe et au moins une séquence hydrophile.
Les lipides amphiphiles non ioniques de l'invention sont préférentiellement choisis parmi :
1/ les tensioactifs siliconés,
2/ les lipides amphiphiles liquides à température inférieure ou égale à 45°C, choisis parmi les esters d'au moins un polyol d'au moins un acide gras comportant au moins une chaîne alkyle en C₈-C₂₂, saturée ou non saturée, linéaire ou ramifiée, et notamment insaturée ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol,
3/ les esters d'acide gras et de sucre et les éthers d'alcool gras et de sucre,
4/ les tensioactifs solides à une température égale à 45°C choisis parmi les esters gras de glycérol, les esters gras de sorbitan et les esters gras de sorbitan oxyéthylénés, les éthers gras éthoxylés et les esters gras éthoxylés,
5/ Les copolymères blocs d'oxyde d'éthylène (A) et d'oxyde de propylène (B), et les mélanges de ces tensioactifs.
1/ Les tensioactifs siliconés utilisables selon l'invention sont des composés siliconés comportant au moins une chaîne oxyéthylénée -OCH₂CH₂- et/ou oxypropylénée - OCH₂CH₂CH₂-, on peut citer ceux décrits dans les documents US-A-5,364,633 et US-A-5,411,744.

De préférence, le tensioactif siliconé utilisé selon la présente invention est un composé de formule (II) : 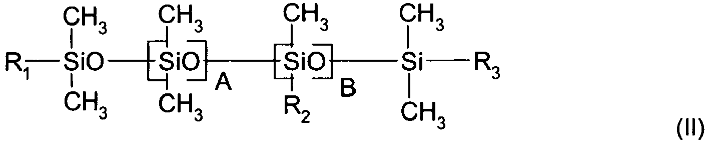 dans laquelle :
R₁, R₂, R₃, indépendamment les uns des autres, représentent un radical alkyle en C₁-C₆ ou
un radical -(CH₂)ₓ-(OCH₂CH₂)_{y}-(OCH₂CH₂CH₂)_{z}-OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (X), le radical alkyle est un radical méthyle, x est un nombre entier allant de 2 à 6 et y est un nombre entier allant de 4 à 30.

On peut citer, à titre d'exemple de tensioactifs siliconés de formule (II), les composés de formule (III) : 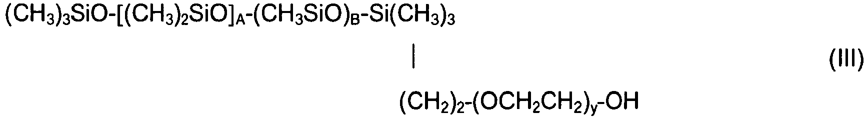 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (II), les composés de formule (IV) :

H-(OCH₂CH₂)_{y}-(CH₂)₃-[(CH₃)₂SiO]_{A}-(CH₂)₃-(OCH₂CH₂)_{y}-OH (IV)

dans laquelle A' et y sont des nombres entiers allant de 10 à 20.

On peut utiliser notamment comme tensioactifs siliconés, ceux commercialisés par la société Dow Corning sous les dénominations DC 5329, DC 7439-146, DC 2-5695 et Q4-3667. Les composés DC 5329, DC 7439-146, DC 2-5695 sont des composés de formule (XI) où respectivement A est 22, B est 2 et y est 12 ; A est 103, B est 10 et y est 12 ; A est 27, B est 3 et y est 12.

Le composé Q4-3667 est un composé de formule (IV) où A est 15 et y est 13.
2/ Les lipides amphiphiles liquides à température inférieure ou égale à 45°C peuvent être notamment choisis parmi :
   - l'isostéarate de polyéthylèneglycol de poids molaire 400 (nom CTFA : PEG-8 Isostearate), vendu sous la dénomination Prisorine 3644 par la société UNICHEMA ;
   - l'isostéarate de diglycéryle, vendu par la société SOLVAY ;
   - le laurate de polyglycérol comportant 2 unités de glycérol (polyglyceryl-2 laurate), vendu sous la dénomination Diglycerin-monolaurate par la société SOLVAY ;
   - l'oléate de sorbitan, vendu sous la dénomination SPAN 80 par la société ICI ;
   - l'isostéarate de sorbitan, vendu sous la dénomination NIKKOL SI 10R par la société NIKKO ;
   - le cocoate d'a-butylglucoside ou le caprate d'a-butylglucoside commercialisés par la société ULICE.
3/ Les esters d'acide gras et de sucre, utilisables comme lipides amphiphiles non ioniques dans la nanoémulsion selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.

Les acides gras en C₈-C₂₂ ou en C₁₄-C₂₂ formant le motif gras des esters utilisables dans la nanoémulsion de l'invention comportent une chaîne alkyle linéaire saturée ou non saturée, ayant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.

On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside.

Les éthers d'alcool gras et de sucre, utilisables comme lipides amphiphiles non ioniques dans la nanoémulsion selon l'invention sont solides à une température inférieure ou égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers utilisables dans la nanoémulsion de l'invention comportent une chaîne alkyle linéaire saturée ou non saturée, ayant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.

A titre d'exemple d'éthers d'alcool gras et de sucre, on peut citer les alkylpolyglucosides tels que le décylglucoside et le laurylglucoside commercialisés par exemple par la société Henkel sous les dénominations respectives de Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidylglucoside, par exemple sous la forme du mélange d'alcools arachidique et béhénique et d'arachidylglucoside, commercialisé sous la dénomination Montanov 202 par la société Seppic.

On utilise plus particulièrement comme lipide amphiphile non ionique de ce type, le monostéarate de sucrose, le distéarate de sucrose, le tristéarate de sucrose et leurs mélanges, le distéarate de méthyl glucose et de polyglycérol-3 et les alkylpolyglucosides.
4/ Les esters gras de glycérol, utilisables comme lipides amphiphiles non ioniques dans la nanoémulsion selon l'invention, solides à une température inférieure ou égale à 45°C, peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol dans la nanoémulsion de l'invention.

Ces esters peuvent être notamment choisis parmi les stéarates, béhénates, arachidates, palmitates et leurs mélanges. On utilise de préférence des stéarates et palmitates.

On peut citer à titre d'exemple de tensioactif utilisable dans la nanoémulsion de l'invention, les monostéarate, distéarate, tristéarate et pentastéarate de décaglycérol (10 unités de glycérol) (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA : Polyglyceryl-2 stearate) tel que le produit vendu par la société Nikko sous la dénomination Nikkol DGMS.

Les esters gras de sorbitan, utilisables comme lipides amphiphiles non ioniques dans la nanoémulsion selon l'invention, solides à une température inférieure ou égale à 45°C, sont choisis notamment dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'oxyde d'éthylène et de préférence de 2 à 40 unités d'oxyde d'éthylène (OE).

Ces esters peuvent être notamment choisis parmi les stéarates, béhénates, arachidates, palmitates, et leurs mélanges. On utilise de préférence des stéarates et palmitates.

On peut citer à titre d'exemple d'ester gras de sorbitan et d'ester gras de sorbitan oxyéthyléné, utilisable dans la nanoémulsion de l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.

Les éthers gras éthoxylés solides à une température inférieure ou égale à 45°C, utilisables comme lipides amphiphiles non ioniques dans la nanoémulsion selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA : Steareth-2), tel que le produit commercialisé sous la dénomination Brij 72 par la société ICI.

Les esters gras éthoxylés solides à une température inférieure ou égale à 45°C, utilisables comme lipides amphiphiles non ioniques dans la nanoémulsion selon l'invention sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société Gattefosse.
5/ Les copolymères blocs d'oxyde d'éthylène et d'oxyde de propylène, utilisables comme lipides amphiphiles non ioniques dans la nanoémulsion selon l'invention peuvent être choisis notamment parmi les copolymères blocs de formule (V) :

   HO(C₂H₄O)x(C₃H₆O)y(C₂H₄O)zH (V)

   dans laquelle x, y et z sont des nombres entiers tels que x+z va de 2 à 100 et y va de 14 à 60, et leurs mélanges, et plus particulièrement parmi les copolymères blocs de formule (V) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (V) avec x=z=6, y=39 (HLB 2) ; le Poloxamer 282 tel que le produit commercialisé par la société ICI sous la dénomination Pluronic L92 de formule (V) avec x=z=10, y=47 (HLB 6) ; et le Poloxamer 124 tel que le produit commercialisé par la société ICI sous la dénomination Pluronic L44 de formule (V) avec x=z=11, y=21 (HLB 16).

Comme lipides amphiphiles non ioniques, on peut aussi citer les mélanges de tensioactifs non ioniques décrits dans le document EP-A-705593 incorporé ici pour référence.

Parmi les lipides amphiphiles non ioniques, on peut utiliser en particulier :
- l'isostéarate de PEG 400 ou PEG-8 Isostéarate (comportant 8 moles d'oxyde d'éthylène),
- l'isostéarate de diglycéryle,
- le monolaurate de polyglycérol comportant 2 unités de glycérol et les stéarates de polyglycérol comportant 10 unités de glycérol,
- l'oléate de sorbitan,
- l'isostéarate de sorbitan,et leurs mélanges.

Les lipides amphiphiles non ionique peuvent être présents dans la nanoémulsion selon l'invention en une teneur allant de **0,2 % à 12 %** en poids, par rapport au poids total de la composition, et de préférence allant de **0,2 % à 8 %** en poids, et préférentiellement allant de **0,2 % à 6 %** en poids.
Les lipides amphiphiles cationiques sont choisis parmi la liste donnée ci après.
Ils sont présents dans les nanoémulsions de l'invention, de préférence, dans des concentrations allant de **0,01 à 6 %** en poids par rapport au poids total de la nanoémulsion et plus particulièrement de **0,2 à 4 %** en poids.

### Huiles :

La phase huileuse de la nanoémulsion selon l'invention comprend au moins une huile. Les huiles pouvant être utilisées dans les nanoémulsions de l'invention sont choisies préférentiellement dans le groupe formé par :
- les huiles d'origine animale ou végétale, formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone, en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- les huiles de synthèse telles que l'huile de parléam, les polyoléfines et les esters d'acides carboxyliques liquides ;
- les huiles minérales telles que l'hexadécane, l'isohexadécane et l'huile de paraffine ;
- les huiles halogénés, notamment des fluorocarbures tels que des fluoramines par exemple la perfluorotributylamine, des hydrocarbures fluorés, par exemple le perfluorodécahydronaphtalène, des fluoroesters et des fluoroéthers ;
- les huiles de silicone volatiles ou non volatiles.

Les polyoléfines utilisables comme huiles de synthèse sont en particulier les poly-α-oléfines et plus particulièrement celles de type polybutène, hydrogéné ou non, et de préférence polyisobutène, hydrogéné ou non.

Les esters d'acides carboxyliques liquides utilisables comme huiles de synthèse, peuvent être des esters d'acides mono-, di-, tri- ou tétra-carboxyliques. Le nombre total de carbone des esters est généralement supérieur ou égal à 10 et de préférence inférieur à 100 et plus particulièrement inférieur à 80. Ce sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant généralement supérieur ou égal à 10. On peut également utiliser les esters d'acides di- ou tri-carboxyliques en C₄-C₂₂ et d'alcools en C₁-C₂₂ et les esters d'acides mono-, di- ou tri-carboxyliques et d'alcools di-, tri-, tétra- ou penta-hydroxylés en C₂-C₂₆.

Parmi les esters cités ci-dessus, on préfère utiliser les palmitates d'alkyle tels que le palmitate d'éthyle, le palmitate d'isopropyle, le palmitate d'éthyl-2-héxyle, le palmitate de 2-octyldécyle ; les myristates d'alkyle tels que le myristate d'isopropyle, le myristate de butyle, le myristate de cétyle, le myristate de 2-octyldodécyle ; les stéarates d'alkyle tel que le stéarate d'hexyle, le stéarate de butyle, le stéarate d'isobutyle ; les malates d'alkyle tels que le malate de dioctyle ; les laurates d'alkyle tels que le laurate d'hexyle et le laurate de 2-hexyldécyle ; l'isononanate d'isononyle ; l'octanoate de cétyle.

Avantageusement, la nanoémulsion selon l'invention contient au moins une huile de poids moléculaire supérieur ou égal à 400, notamment allant de 400 à 10 000, mieux allant de 400 à 5000, ou encore allant de 400 à 2500. Les huiles de poids moléculaire supérieur ou égal à 400 peuvent être choisies parmi les huiles d'origine animale ou végétale, les huiles minérales, les huiles de synthèse et les huiles de silicone, et leurs mélanges. Comme huiles de ce type, on peut citer par exemple le palmitate d'isocétyle, le stéarate d'isocétyle, l'huile d'avocat, l'huile de jojoba.

Les nanoémulsions conformes à l'invention comportent une quantité de phase huileuse (huile et autres corps gras hormis le lipide amphiphile) allant de préférence, de 2 à 40 % en poids par rapport au poids total de la nanoémulsion et plus particulièrement de 4 à 30 % en poids et préférentiellement de 4 à 20 % en poids.

La phase huileuse et les lipides amphiphiles (amphiphiles non ioniques et ioniques) sont de préférence présents dans la nanoémulsion selon l'invention selon un rapport pondéral de la quantité de phase huileuse sur la quantité de lipide amphiphile allant de 3 à 10 et préférentiellement allant de 3 à 6. On entend ici par "quantité de phase huileuse" la quantité totale des constituants de cette phase huileuse sans inclure la quantité de lipide amphiphile. Les nanoémulsions conformes à la présente invention peuvent contenir, en complément des dérivés d'urée de formule (I) décrits précédemment, des solvants, notamment pour améliorer, si nécessaire, la transparence de la composition.

Ces solvants sont choisis de préférence dans le groupe formé par :
- les alcools inférieurs en C₁-C₈ tels que l'éthanol ;
- les glycols tels que la glycérine, le propylèneglycol, le 1,3-butylèneglycol, le dipropylèneglycol, les polyéthylèneglycols comportant de 4 à 16 unités d'oxyde d'éthylène et de préférence de 8 à 12 ;
- les sucres tels que le glucose, le fructose, le maltose, le lactose, le sucrose.

Ces solvants peuvent être utilisés en mélange. Lorsqu'ils sont présents dans la nanoémulsion de l'invention, ils peuvent être utilisés à des concentrations allant de préférence de 0,01 à 30 % en poids par rapport au poids total de la nanoémulsion, et mieux de 5 à 20 % en poids par rapport au poids total de la nanoémulsion. La quantité d'alcool(s) et/ou de sucre(s) va de préférence de 5 à 20 % en poids par rapport au poids total de la nanoémulsion et la quantité de glycol(s) va de préférence de 5 à 15 % en poids par rapport au poids total de la nanoémulsion.

### Procédé de préparation :

Le procédé de préparation d'une nanoémulsion telle que définie ci-dessus consiste à mélanger la phase aqueuse contenant le dérivé d'urée et la phase huileuse, sous agitation vive, à une température allant de 10 °C à 80 °C, à effectuer une étape d'homogénéisation haute pression à une pression supérieure à 5.10⁷ Pa et à ajouter éventuellement le polymère utilisé. Selon un mode préféré de réalisation de l'invention, on effectue ensuite encore une étape d'homogénéisation haute pression à une pression supérieure à 5.10⁷ Pa. L'homogénéisation haute pression est réalisée de préférence à une pression allant de 6.10⁷ à 18.10⁷ Pa. Le cisaillement va de préférence de 2.10⁶ s⁻¹ à 5.10⁸ s⁻¹ et mieux de 1.10⁸ s⁻¹ à 3.10⁸ s⁻¹ (s⁻¹ signifie seconde⁻¹). Un tel procédé permet de réaliser des nanoémulsions compatibles avec des composés actifs thermosensibles, et pouvant contenir des huiles et notamment des parfums qui renferment des corps gras, sans les dénaturer.

### Nanocapsules

Les nanocapsules relatives à l'invention sont celles décrites dans les demandes de brevet EP 0 447 318, EP 0 557 489, EP 0 780 115, EP 1 025 901, EP 1 029 587, EP 1 034 839, EP 1 414 390, FR 2 830 776, EP 1 342 471, FR 2 848 879 et FR 04/50057.

Les nanocapsules sont des particules Core-Shell ayant un coeur huileux et une coque polymérique. Les différentes demandes citées plus haut portent sur différentes familles de polymères et différents procédés pour les obtenir. La taille des capsules est toujours inférieure à 1 µm et il est possible d'avoir des tailles inférieures à 80 nm. Ces particules peuvent être enrobées par une phase cristal liquide lamellaire le plus souvent constituée d'une lécithine ou d'un diméthicone copolyol. L'enrobage devant être constitué d'un lipide amphiphile apte à former spontanément une phase cristal liquide lamellaire au contact de l'eau. C'est à ce lipide amphiphile apte à former une phase lamellaire que sera ajouté le tensioactif cationique qui conférera à la particules (la nanocapsule) un potentiel zeta positif. Le rapport pondéral entre le lipide amphiphile formant la phase lamellaire et le tensioactif cationique sera compris entre 99/1 et 75/25.

Les tensioactifs cationiques utilisables sont ceux listés ci-après.

### Particules organiques

Les particules organiques de l'invention sont des nanosphères, pleines, sans cavité interne formée par différents procédés (dispersion dans l'eau, nanoprécipitation, microémulsion...) et composées d'au moins un polymère ou d'au moins un copolymère ou d'un mélange de ceux ci. La particule est cationique, avec le potentiel zeta défini précédemment, soit parce que le ou les polymères ou copolymères sont cationiques, soit parce que non ioniques et on utilise un tensioactif cationique tel que décrit ci-après. Par rapport au polymère, le taux de tensioactif cationique sera compris entre 0 et 25%.

### Particules inorganiques

Les particules inorganiques cationiques de l'invention peuvent être, à titre d'exemple, à base de silice, TiO2, ZnO, d'alumine... A titre d'exemple, on citera les particules d'alumine en dispersion colloïdale dans l'eau comme les Nanomer 2 de Nalco. Clariant et Grace, proposent également des particules de ce type.

Tensioactifs cationiques utilisables pour la préparation des particules cationiques de

### l'invention

Les tensioactifs cationiques qui peuvent être utilisés selon l'invention sont listés ci-après, cette liste est non limitative.

Les lipides amphiphiles cationiques sont choisis, de préférence, dans le groupe formé par les sels d'ammonium quaternaire, les amines grasses et leurs sels.

Les sels d'ammonium quaternaires sont par exemple :
- ceux qui présentent la formule générale (IV) suivante : 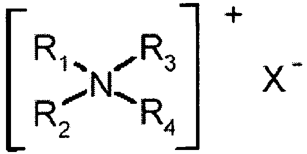 dans laquelle les radicaux R1 à R4, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C2-C6), alkylamide, alkyl(C12-C22)amido alkyle(C2-C6), alkyl(C12-C22)acétate, hydroxyalkyle, comportant environ de 1 à 30 atomes de carbone; X est un anion choisi dans le groupe des halogénures, phosphates, acétates, lactates, alkyl(C2-C6)sulfates, alkyl-ou-alkylarylsulfonates,
- les sels d'ammonium quaternaire de l'imidazolinium, comme par exemple celui de formule (V) suivante : 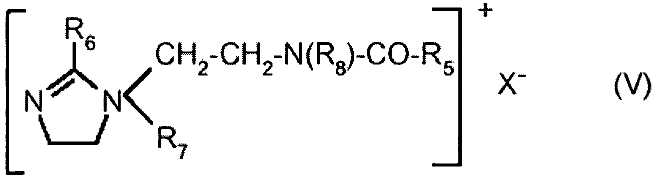 dans laquelle R5 représente un radical alcényle ou alkyle comportant de 8 à 30 atomes de carbone par exemple dérivés des acides gras du suif, R6 représente un atome d'hydrogène, un radical alkyle en C1-C4 ou un radical alcényle ou alkyle comportant de 8 à 30 atomes de carbone, R7 représente un radical alkyle en C1- C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates. De préférence, R5 et R6 désignent un mélange de radicaux alcényle ou alkyle comportant de 12 à 21 atomes de carbone par exemple dérivés des acides gras du suif, R7 désigne méthyle, R8 désigne hydrogène. Un tel produit est par exemple commercialisé sous la dénomination «REWOQUAT W 75» par la société REWO,
- les sels de diammonium quaternaire de formule (VI) : 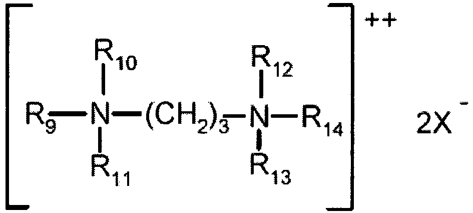 dans laquelle R9 désigne un radical aliphatique comportant environ de 16 à 30 atomes de carbone, R10, R11, R12, R13 et R14 , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester
Les sels d'ammonium quaternaire contenant au moins une fonction ester utilisables selon l'invention sont par exemple ceux de formule (VII) suivante : 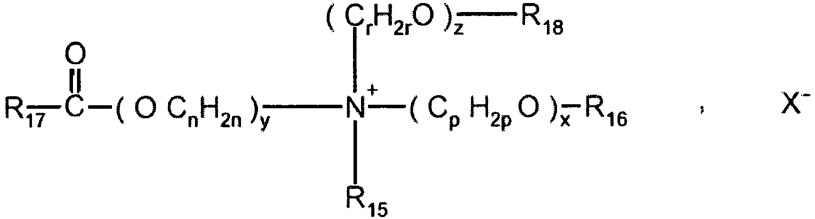 dans laquelle :
- R15 est choisi parmi les radicaux alkyles en C1-C6 et les radicaux hydroxyalkyles ou dihydroxyalkyles en C1-C6 ;
- R16 est choisi parmi :
- le radical 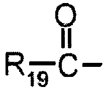
- les radicaux R20 hydrocarbonés en C1-C22 linéaires ou ramifiés, saturés ou insaturés,
- l'atome d'hydrogène,
- R18 est choisi parmi :
- le radical 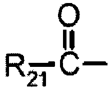
- les radicaux R22 hydrocarbonés en C1-C6 linéaires ou ramifiés, saturés ou insaturés,
- l'atome d'hydrogène,
- R17, R19 et R21, identiques ou différents, sont choisis parmi les radicaux hydrocarbonés en C7-C21, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ; sous réserve que la somme x + y + z vaut de 1 à 15, que lorsque x vaut 0 alors R16 désigne R20 et que lorsque z vaut 0 alors R18 désigne R22.
Les radicaux alkyles R15 peuvent être linéaires ou ramifiés et plus particulièrement linéaires. De préférence R15 désigne un radical méthyle, éthyle, hydroxyéthyle ou dihydroxypropyle et plus particulièrement un radical méthyle ou éthyle. Avantageusement, la somme x + y + z vaut de 1 à 10.
Lorsque R16 est un radical R20 hydrocarboné, il peut être long et avoir de 12 à 22 atomes de carbone ou court et avoir de 1 à 3 atomes de carbone.
Lorsque R18 est un radical R22 hydrocarboné, il a de préférence 1 à 3 atomes de carbone. Avantageusement, R17, R19 et R21, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.
De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.
L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X- est encore plus particulièrement le chlorure ou le méthylsulfate.

On utilise plus particulièrement les sels d'ammonium de formule (VII) dans laquelle :
- R15 désigne un radical méthyle ou éthyle,
- x et y sont égaux à 1 ;
- z est égal à 0 ou 1 ;
- n, p et r sont égaux à 2 ;
- R16 est choisi parmi :
- le radical 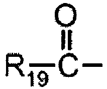
- les radicaux méthyle, éthyle ou hydrocarbonés en C14-C22
- l'atome d'hydrogène ;
- R18 est choisi parmi :
- le radical 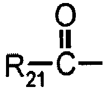
- l'atome d'hydrogène ;
R17, R19 et R21, identiques ou différents, sont choisis parmi les radicaux hydrocarbonés en C13-C17, linéaires ou ramifiés, saturés ou insaturés et de préférence parmi les radicaux alkyles et alcényle en C13-C17, linéaires ou ramifiés, saturés ou insaturés. Avantageusement, les radicaux hydrocarbonés sont linéaires.

On peut citer par exemple les composés de formule (VII) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.
Lorsque le composé contient plusieurs radicaux acyles, ces derniers peuvent être identiques ou différents.
Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.
De tels composés sont par exemple commercialisés sous les dénominations DEHYQUART par la société HENKEL, STEPANQUAT par la société STEPAN, NOXAMIUM par la société CECA, REWOQUAT WE 18 par la société REWOWITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60 % de méthylsulfate de diacyloxyéthyl hydroxyéthyl méthyl ammonium et 15 à 30 % de méthylsulfate de triacyloxyéthyl méthyl ammonium, les radicaux acyles ayant de 14 à 18 atomes de carbone et provenant d'huile de palme éventuellement partiellement hydrogénée. On peut aussi utiliser les sels d'ammonium contenant au moins une fonction ester décrits dans les brevets US-A-4874554 et US-A-4137180.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Selon l'invention, le chlorure ou le bromure de béhényltriméthylammonium et le CTAB (cétyltrimethylammonium bromine) sont les sels d'ammonium quaternaires les plus particulièrement préférés.

Les amines grasses de l'invention correspondent à la formule générale : 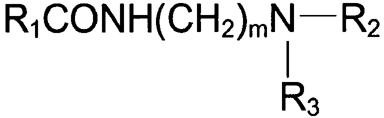
- R₁ est une chaîne hydrocarbonée saturée ou non, ramifiée ou non, ayant entre 8 et 30 atomes de carbone et de préférence entre 10 et 24.
- R₂ et R₃ sont sélectionnés indépendamment parmi hydrocarbonées saturées ou non, ramifiées ou non, ayant entre 1 et 10 atomes de carbone et de préférence entre 1 et 4.
- R₂ et R₃ peuvent également, toujours indépendamment l'un de l'autre correspondre à un atome d'hydrogène H .
- M est compris entre 1 et 10 et de préférence entre 1 et 5.
A titre d'exemple non limitatif, on citera : la stearylamine, le Stearate aminoethylethanolamide, Stearyl diethanolamide, Stearate diethylenetriamine, la stearamidopropyldimethylamine, la stearamidopropyldiethylamine, la stearamidoethyldiethylamine, la stearamidoethyldimethylamine, la palmitamidopropyldimethylamine, la palmitamidopropyldiethylamine, la palmitamidoethyldiethylamine, la palmitamidoethyldimethylamine, la behenamidopropyldimethylamine, la behenamidopropyldiethylmine, la behenamidoethyldiethylamine, la behenamidoethyldimethylamine, la arachidamidopropyldimethylamine, l'arachidamidopropyldiethylamine, l'arachidamidoethyldiethylamine, l'arachidamidoethyldimethylamine.
On peut citer comme amine grasse commercialement disponible, l'Incromine BB de Croda, l'Amidoamine MSP de Nikkol, les Lexamine de Inolex.
Comme autres amines grasses, on citera, à titre d'exemple, la stearylamine, le Stearate aminoethylethanolamide, la Stearyl diethanolamide, le Stearate diethylenetriamine que, entre autres, Sabo commecrcialise avec la série des Sabomina.
On citera également les acétates amines grasses comme la série des Acetamine de Kao Corp.
Ces amines grasses peuvent être également ethoxylées comme les Berol 380, 390, 453 et 455, les Ethomeen de Akzo Nobel ou les Marlazin L10, OL2, OL20, T15/2, T50 de Condea Chemie.

Les particules de la présente invention peuvent être introduites dans tous supports galéniques à visée cosmétique, dermatologique et ophtalmique. A titre d'exemple, on citera les lotions, les serums, les gels et tous les type d'émulsions.

Les compositions selon l'invention peuvent présenter toutes les formes galéniques normalement utilisées pour une application topique, par exemple sous forme de solutions, de gels, de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Les compositions selon l'invention peuvent comprendre en outre tout additif usuellement utilisé dans le domaine cosmétique ou pharmaceutique.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées.
En particulier, on veillera à ce que cette introduction ne nuise pas à la stabilité des particules cationiques associées au siRNA.

La composition selon l'invention pourra en particulier contenir des actifs cosmétiques ou pharmaceutiques. De préférence, il s'agira d'agents dépigmentants, de filtres solaires.

Les agents dépigmentants susceptibles d'être incorporés dans la composition comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Les agents filtrant le rayonnement ultra violet peuvent être choisis parmi les filtres UV organiques ou les agents filtrant les radiations UV minéraux.

Les filtres UV organiques conformes à l'invention peuvent être hydrosolubles, liposolubles ou insolubles dans les solvants usuels cosmétiques. Ils sont choisis notamment parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4,367,390, EP 863 145, EP 517 104, EP 570 838, EP 796 851, EP 775 698, EP 878 469 et EP 933 376 ; les dérivés de la benzophénone notamment ceux décrits dans les demandes EP 1 046 391 et DE10012408; les dérivés de β, β'-diphénylacrylate, les dérivés de benzotriazole, les dérivés de benzimidazole ; les imadazolines ; les dérivés bis-benzoazolyle tels que décrits dans les brevets EP 669 323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB 2303549, DE 19726184 et EP 893 119 ; les polymères filtres et silicones filtres tels que ceux décrits notamment dans la demande WO93/04665 ; les dimères dérivés d'α-alkylstyrène tels que ceux décrits dans la demande de brevet DE19855649 ; les dérivés de 4,4-diarylbutadiène tels que ceux décrits dans les demandes de brevet EP 0 967 200 et DE19755649.

Les agents filtrant minéraux sont généralement des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP 0 518 772 et EP 0 518 773.
Les agents filtrants les radiations conformes à l'invention sont généralement présents dans les compositions selon l'invention dans des proportions allant de 0,1 à 20 % en poids par rapport au poids total de la composition, et de préférence allant de 0,2 à 15 % en poids par rapport au poids total de la composition.

La présente demande décrit également à l'utilisation d'au moins un oligonucléotide d'ARN double brin de séquence choisie parmi les SEQ. ID. n°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 et 48 pour inhiber l'expression de la tyrosinase.

Selon un autre de ses objets, la présente invention se rapporte également à un oligonucléotide d'ARN double brin de séquence choisie parmi les SEQ. ID. n°2, 4, 5, 7, 8, 13, 14, 16, 23, 25, 29, 32, 35, 37, 40, 42, 43, 46 ou 47, destiné à être utilisé pour inhiber l'expression de la tyrosinase.

En particulier, les oligonucléotides selon l'invention sont utiles comme agent blanchissant et/ou comme agent anti-brunissementde la peau.
Les oligonucléotides d'ARN double brin selon l'invention peuvent également être utilisés pour la préparation de composition topique, cosmétique ou dermatologique, destinée à diminuer la synthèse de la mélanine, notamment, en vue de traiter l'hyperpigmentation, des tâches et dyschromies pigmentaires ou pour le blanchiment des follicules pileux, les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc), à défaut de pouvoir repigmenter la peau lésée, on achève de dépigmenter les zones de peau normale résiduelle pour donner à l'ensemble de la peau une teinte blanche homogène.

La présente demande décrit un procédé cosmétique pour blanchir et/ou éclaircir le teint et/ou uniformiser la couleur d'une peau brunie comprenant l'application topique d'au moins un oligonucléotide d'ARN double brin de séquence choisie parmi la SEQ. ID. n°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 et 48.

Un autre objet de la présente invention se rapporte à un procédé cosmétique pour blanchir et/ou éclaircir le teint et/ou uniformiser la couleur d'une peau brunie comprenant l'application topique d'au moins un oligonucléotide d'ARN double brin de séquence choisie parmi la SEQ. ID. n°2, 4, 5, 7, 8, 13, 14, 16, 23, 25, 29, 32, 35, 37, 40, 42, 43, 46 ou 47.
De préférence, l'oligonucléotide est formulé dans une composition topique à une concentration inférieure ou égale à 1 nM.
De façon encore préférentielle, l'oligonucléotide est associé à une particule cationique d'une taille inférieure ou égale à 1 µm, de potentiel zeta compris entre 10 et 80 mV choisie parmi les micelles de tensioactifs, les micelles de polymères blocs, les liposomes de tensioactifs non ionique et cationiques, les niosomes, les oléosomes, les particules de nanoémulsions, les nanocapsules, les particules organiques ou les particules inorganiques, telles que décrites ci-avant.
A des fins de soin cosmétique, dermatologique, ophtalmique ou pharmaceutique, on cherche à éviter les méthodes invasives telles que les injections sous cutanées ou oculaires. Les particules cationiques associées au siRNA selon la présente invention peuvent être appliquées topiquement sur la peau, les muqueuses ou dans l'oeil, en suspension ou incorporées dans un support cosmocentrique acceptable, comme des lotions, sérum, gels émulsionnés ou non, des émulsions huile/eau, eau/huile, multiples, les microémulsions... Pour favoriser la pénétration des particules associées au siRNA, on pourra modifier la perméabilité de la peau, ainsi, il est possible de contrôler la perméabilité de la peau par la mesure de la PIE (perte insensible en eau) avec un Tewametre TM210 ou un Dermalab - Cortex technology.
On pourra utiliser, à titre d'exemple, les méthodes suivantes :
- stripping (cornéodisque, « vernis »), peeling chimique ou dermabrasion mécanique avant application de la composition selon l'invention ;
- pré-traitement par un mélange d'un ou plusieurs solvants ayant un effet délipidant ;
- nettoyage de la peau par un produit moussant détergent ;
- pré et/ou post traitement occlusif soit par exemple en recouvrant la surface de la peau à traiter d'une membrane synthétique étanche (Blenderm par exemple) soit par l'application d'une couche de vaseline. Cela a pour effet de bloquer la PIE naturelle de la peau et de provoquer une sur-hydratation des lipides épidermiques qui deviennent ainsi plus perméable. Après l'application de la composition de la présente invention, on pourra également utiliser des méthodes connues de l'homme du métier favorisation la pénétration de molécules au sein de la peau telles que, par exemple, la iontophorèse ou l'électroporation.

Dans le cas d'un modèle de peau *in vitro,* on pourra appliquer les traitements suivants :
- avant dépôt de la composition selon l'invention, on pourra réduire le taux de Ca2+ soit par le changement du milieu (0,10-0,75 mM au lieu de 1,5 mM) soit par l'ajout d'un chélatant. Cela a pour effet de diminuer l'adhésion intermembranaire, augmentant ainsi la perméabilité de la peau (*Effects of extra- and intracellular calcium concentration on DNA replication, latéral growth, and differentiation of human epidermal cells in culture.* Virchows Arch B Cell Pathol Incl Mol Pathol. 1990;59(1): 17-25) *;*
- en utilisant la iontophorèse, éléctroporation et toutes méthodes connues permettant de modifier la perméabilité du modèle.

### EXEMPLE 1 - FORMULATIONS

### Micelles cationiques

- Exemple 1 : Octyl β Glucoside / cetyl triammonium bromide au ratio molaire de 5/1
   E1 : 100 mM dans l'eau distillée
   E2 : 50 mM dans l'eau distillée
   E3 : 25 mM dans l'eau distillée
   E4 : 12,5 mM dans l'eau distillée
   Les 2 tensio actifs (non ionique + cationique) sont solubilisés dans l'eau distillée. Une suspension du siRNA de SEQ. ID. 1 (20 µM) est ajoutée entre 1:1 et 1:9 volume (siRNA : micelles) réduisant ainsi la concentration micellaire en proportion.
- Exemple 2 : Micelles de décyl β glucoside / behenyl triammonium chloride au ratio molaire de 5/1 dans l'eau distillée. Les mêmes concentrations que dans l'exemple précédent sont réalisées.

Ces 2 suspensions peuvent être appliquées sur la peau pour le traitement des tâches et dyschromies pigmentaires ou pour le blanchiment des follicules pileux.

### Liposomes cationiques

### - Exemple 1 : vésicules « fluides »

| | |
|---|---|
| Isostéarate de PEG 400 | 5.5% |
| Behenyl triammonium chloride | 0.5% |
| Eau distillée | qsp 100 |

### - Exemple 2 : vésicules « rigides »

| | |
|---|---|
| Palmitate de sorbitan | 2.75% |
| Cholestérol | 2.75% |
| Behenyl triammonium chloride | 0.5% |
| Eau distillée | qsp 100 |

Ces exemples de suspensions de liposomes sont réalisés par dialyse. Les lipides constitutifs des vésicules sont solubilisés dans une solution aqueuse d'octyl β glucoside. Cette solution est ensuite dialysée contre de l'eau pendant 72h.

La suspension de siRNA SEQ. ID. n°4 est alors ajoutée, ramenant la concentration en vésicule autour de 3% de lipide. Cette suspension de liposomes non ioniques peut être appliquée sur la peau pour le traitement des tâches et dyschromies pigmentaires ou pour le blanchiment des follicules pileux.

### Oléosomes cationiques

### - Phase huile

| | |
|---|---|
| Mono-di stéarate de sucrose commercialisé par Stéarainerie Dubois | 0.45% |
| Stéarate de sorbitane 40E (Tween 61 Uniquema) | 0.30% |
| Béhényl triammonium chloride | 0.21% |
| Acétate de Vit E | 0.5% |
| Huile de jojoba | 0.5% |
| Stéaryl heptanoate | 1% |
| Huile se silicone volatile | 1% |
| Glycéride de Vit F | 0.5% |
| Conservateur | 0.02% |
| BHT | 0.01% |

### - Phase aqueuse

| | |
|---|---|
| Eau distillée | qsp 100 |
| Conservateur | 0.1% |

Cette dispersion est réalisée par homogénéisation haute pression afin d'avoir une taille de particule d'environ 170 nm. Est ajoutée ensuite, une suspension de siRNA SEQ. ID. n°16 (20 µM) à des ratii allant de 1:1 à 1:20 (siRNA/Oléosomes). Le siRNA va alors se complexer à la surface des particules.

### Nanocapsules cationiques

### - Phase organique

| | |
|---|---|
| Polycaprolactone (MW :50000) | 1% |
| Vit E | 1% |
| Dimethicone copolyol DC2 5695 (Dow Corning) | 0.5% |
| Béhényl triammonium chloride | 0.21% |
| Acétone | 200 ml |

### - Phase aqueuse

| | |
|---|---|
| Pluronic F68 | 0.5% |
| Eau distillée | 200ml |

La phase organique est introduite sous agitation dans la phase aqueuse. L'acétone et 100 ml de phase aqueuse sont ensuite évaporés pour obtenir la suspension de nanocapsules dont la taille est de 220 nm. Est ajoutée ensuite, une suspension de siRNA SEQ. ID. n° 37 (20 µM) à des ratii allant de 1:1 à 1:20 (siRNA/nanocapsules). Le siRNA va alors se complexer à la surface des particules.

### Nanoparticules organiques

### - Phase organique

| | |
|---|---|
| Polyethylène adipate (Scientific Polymer products) | 2% |
| Dimethicone copolyol DC2 5695 (Dow Corning) | 0.5% |
| Béhényl triammonium chloride | 0.21% |
| Acétone | 200 ml |

### - Phase aqueuse

| | |
|---|---|
| Pluronic F68 | 0.5% |
| Eau distillée | 200ml |

La phase organique est introduite sous agitation dans la phase aqueuse. L'acétone et 100 ml de phase aqueuse sont ensuite évaporés pour obtenir la suspension de nanoparticules dont la taille est de 180 nm. Est ajoutée ensuite, une suspension de siRNA SEQ. ID. n° 40 (20 µM) à des ratii allant de 1:1 à 1:20 (siRNA/nanoparticules). Le siRNA va alors se complexer à la surface des particules.

### Nanoémulsion cationiques

### - Phase Huile

| | |
|---|---|
| Isostéarate de PEG 400 commercialisé par Uniqema | 1% |
| Béhényl triammonium chloride | 1% |
| Huile d'avocat | 1% |
| Huile de Jojaba | 3% |
| Cyclopentaméthylsiloxane | 2% |

### - Phase aqueuse

| | |
|---|---|
| Eau distillée | 30% |
| Dipropylène glycol | 10% |

### - Phase de dilution

| | |
|---|---|
| Eau distillée | qsp 100% |
| Conservateur | 0.1% |

Une émulsion est réalisée en dispersant la phase huileuse sur la phase aqueuse sous très vive agitation. La suspension obtenue est ensuite homogénéisée plusieurs fois à l'aide d'un homogénéisateur très haute pression, à une pression d'environ 1200 b. La taille des particules est de l'ordre de 50 nm et la suspension est transparente. La phase de dilution est ensuite ajoutée.

Comme dans les cas précédent, est ajoutée ensuite, une suspension de siRNA SEQ. ID. n° 42 (20µM) à des ratii allant de 1:1 à 1:20 (siRNA/nanoémulsion). Le siRNA va alors se complexer à la surface des particules.

### EXEMPLE 2 - Mesure de l'activité des siRNA selon l'invention

L'efficacité des 47 siRNA de séquence SEQ ID n°1 à 47 selon l'invention a été évaluée dans le test « pSCREEN-iT™ system » développé par Invitrogen mis au point pour évaluer l'efficacité sur l'inhibition de la tyrosinase (GenBank accession number M27160).

### Protocole experimental du test:

- Ensemencement de 2.10⁴ cellules A549/puits dans une plaque 48 puits et cultivées une nuit.
- Co-transfection en triplicate pendant 5h en utilisant 2 µg/ml de Lipofectamine 2000 (Invitrogen) complexée avec:
   - Tyrosinase pScreen-iT™ vector couplé au gène LacZ- 10 ng/ puits
   - Luciferase reporter plasmid - 20 ng/ puits
   - 1 nM Stealth™ RNA (séquences données dans le tableau I)
- Les cellules sont lysées 24h après la transfection et les activités β-Galactosidase (β-Gal) et Luciferase (Luc) sont mesurées. Le rapport β-Gal/Luc détermine le pourcentage d'inhibition de la tyrosinase.

Les résultats obtenus sont présentés dans le Tableau II.

**Tableau II : dosage de l'efficacité des 47 oligonucléotides d'ARN double brin sur la dégradation du mRNA de la tyrosinase (GenBank accession number M27160).**

| **Oligonucléotide (SEQ. ID. n°)** | **% Inhibition** | **Oligonucléotide (SEQ. ID. n°)** | **% Inhibition** |
|---|---|---|---|
| 1 | 97,50% | 22 | 77,91% |
| 2 | 98,57% | 23 | 97,29% |
| 3 | 86,95% | 24 | 90,50% |
| 4 | 97,95% | 25 | 95,17% |
| 5 | 94,17% | 26 | 90,60% |
| 6 | 77,68% | 27 | 87,11% |
| 7 | 95,86% | 28 | 91,58% |
| 8 | 97,50% | 29 | 96,00% |
| 9 | 87,14% | 30 | 90,39% |
| 10 | 91,54% | 31 | 10,38% |
| 11 | 88,44% | 32 | 97,93% |
| 12 | 89,50% | 33 | 84,79% |
| 13 | 98,15% | 34 | 64,43% |
| 14 | 97,87% | 35 | 97,71% |
| 15 | 36,02% | 36 | 92,12% |
| 16 | 97,78% | 37 | 97,07% |
| 17 | 88,54% | 38 | 9,55% |
| 18 | 81,11% | 39 | 90,22% |
| 19 | 40,78% | 40 | 97,44% |
| 20 | 83,33% | 41 | 88,11% |
| 21 | 92,64% | 42 | 98,71% |
| 43 | 99,10% | 46 | 98,64% |
| 44 | 83,54% | 47 | 94,14% |
| 45 | 89,05% | | |

Dans un second temps, un effet dose a été effectué sur les 20 séquences les plus efficaces cad avec une efficacité supérieure à 91 % à 1 nM. Les résultats sont présentés dans le tableau III.

**Tableau III : dosage de l'efficacité des 20 séquences sélectionnées par effet dose de 0.0016 nM à 1 nM.**

| **Oligonucléotide (SEQ. ID. n°)** | **1 nM** | **0.25 nM** | **0.063 nM** | **0.016 nM** |
|---|---|---|---|---|
| 1 | 97,37% | 94,34% | 89,47% | 72,07% |
| 2 | 97,98% | 95,64% | 89,34% | 66,68% |
| 4 | 97,93% | 95,22% | 90,24% | 57,92% |
| 5 | 91,33% | 79,88% | 61,47% | 18,40% |
| 7 | 96,86% | 88,97% | 76,45% | 38,34% |
| 8 | 97,84% | 90,76% | 81,12% | 38,57% |
| 13 | 98,13% | 95,73% | 91,11% | 62,56% |
| 14 | 97,59% | 87,22% | 68,77% | 33,87% |
| 16 | 97,95% | 94,92% | 83,40% | 56,25% |
| 23 | 96,05% | 87,42% | 62,32% | 29,97% |
| 25 | 93,45% | 90,50% | 78,36% | 46,80% |
| 29 | 96,42% | 88,24% | 70,64% | 31,15% |
| 32 | 96,88% | 90,69% | 80,33% | 35,04% |
| 35 | 97,42% | 94,84% | 88,34% | 54,29% |
| 37 | 96,38% | 92,46% | 88,24% | 53,92% |
| 40 | 97,65% | 95,08% | 91,59% | 60,64% |
| 42 | 98,25% | 96,54% | 92,58% | 53,46% |
| 43 | 98,46% | 95,60% | 89,21% | 38,85% |
| 46 | 98,77% | 94,81% | 84,23% | 48,74% |
| 47 | 94,60% | 76,71% | 52,94% | 30,18% |

Les siRNA selon la présente invention montrent une efficacité supérieure à 94% à 1 nM pour les 20 séquences les plus efficaces. La dose la plus faible testée est 0.016 nM pour une efficacité de 72% sur la dégradation de l'ARNm codant pour la tyrosinase.

### EXEMPLE 3 - Evaluation d'une éventuelle réponse type interféron provoquée par les siRNA selon l'invention

Il a été vérifié que les siRNA objets de la présente invention n'induisaient pas de réponse type interféron. Pour cela, l'expression des gènes OAS-1 et IFIT-1 (interferon-inducible tetratricopeptide repeat domain) connus pour être induits par l'interféron *(*Wieland SF et al., Searching for Interferon-Induced Genes That Inhibit Hepatitis B Virus Replication in Transgenic Mouse Hepatocytes, J. of Virology 2003 ; 77:1227-1236*.* Persengiev SP et al., Nonspecific, concentration-dependent stimulation and repression of mammalian gene expression by small interfering RNAs (siRNAs). RNA 2004;10: 12-18*.* Bridge AJ et al., Induction of an interferon response by RNAi vectors in mammalian cells. Nature Genet. 2003; 34: 263-264*.* Scacheri PC et al., Short interfering RNAs can induce unexpected and divergent changes in the levels of untargeted proteins in mammalian cells. Proc. Natl. Acad. Sci. USA 2004, 101:1892-1897*.* Sledz CA et al., Activation of the interferon system by short-interfering RNAs. Nat Cell Biol. 2003;9: 834-9*.* Patzwahl R et al., Enhanced expression of interferon-regulated genes in the liver of patients with chronic hepatitis C virus infection: detection by suppression-subtractive hybridization. J. Virol. 2001; 75: 1332-1338.*)* a été mesurée par PCR quantitative (qPCR) selon le protocole décrit dans le manuel du kit « BLOCK-iT™ RNAi Stress Response Control Kit (Human) For monitoring interferon-mediated stress response to double-stranded RNA in human cells » commercialisé par Invitrogen.

### Mesure du niveau d'expression du gène hOAS-1 :

Le niveau d'expression du gène hOAS-1 a été rapporté a celui du gène de référence GAPDH.

| **Echantillon** | **hOAS-1 / GAPDH** | **Stdev** |
|---|---|---|
| dsRNA de SEQ. ID. n°1 - 1 nM | 0,608 | 0,046 |
| dsRNA de SEQ. ID. n°2 - 1 nM | 0,668 | 0,292 |
| dsRNA de SEQ. ID. n°4 - 1 nM | 0,608 | 0,070 |
| dsRNA de SEQ. ID. n°13 - 1 nM | 1,232 | 0,866 |
| dsRNA de SEQ. ID. n°46 - 1 nM | 0,911 | 0,221 |
| dsRNA de SEQ. ID. n°1 - 50 nM | 1,080 | 0,500 |
| Séquence de controle à 1 nM | 2,141 | 1,336 |
| Contrôle positif (long dsRNA de 1 kb) | 24,905 | 6,431 |
| dsRNA de SEQ. ID. n°1 - 0,1 nM | 0,856 | 0,534 |
| dsRNA de SEQ. ID. n°2 - 0,1 nM | 0,866 | 0,300 |
| dsRNA de SEQ. ID. n°4 - 0,1 nM | 0,966 | 0,568 |
| dsRNA de SEQ. ID. n°13 - 0,1 nM | 1,238 | 0,451 |
| dsRNA de SEQ. ID. n°46 - 0,1 nM | 1,262 | 0,328 |
| Séquence de contrôle à 50 nM | 0,807 | 0,404 |
| Séquence de contrôle à 0,1 nM | 1,949 | 1,502 |

Résultat : seul le contrôle positif (long dsRNA de 1 kb) induit une augmentation significative de l'expression du gène hOAS-1. Les séquences testées (SEQ. ID. n°1, 2, 4, 13 et 46) n'augmentent pas de façon significative l'expression du gène hOAS-1.

**Mesure du niveau d'expression du gène hIFIT-1**

| **Echantillon** | **cycle seuil** | **Stdev** |
|---|---|---|
| dsRNA de SEQ. ID. n°1 - 1 nM | 36,15 | 0,20 |
| dsRNA de SEQ. ID. n°2 - 1 nM | 32,77 | 1,85 |
| dsRNA de SEQ. ID. n°4 - 1 nM | 35,98 | 0,88 |
| dsRNA de SEQ. ID. n°13 - 1 nM | 35,41 | 1,88 |
| dsRNA de SEQ. ID. n°46 - 1 nM | 34,42 | 3,24 |
| dsRNA de SEQ. ID. n°1 - 50 nM | 34,16 | 3,02 |
| Séquence de contrôle à 1 nM | 31,30 | 1,98 |
| Contrôle positif (long dsRNA de 1 kb) | 25,70 | 0,79 |
| dsRNA de SEQ. ID. n°1 - 0,1 nM | 32,47 | 3,07 |
| dsRNA de SEQ. ID. n°2 - 0,1 nM | 35,38 | 1,41 |
| dsRNA de SEQ. ID. n°4 - 0,1 nM | 34,86 | 1,86 |
| dsRNA de SEQ. ID. n°13 - 0,1 nM | 33,22 | 2,33 |
| dsRNA de SEQ. ID. n°46 - 0,1 nM | 33,89 | 2,03 |
| Séquence de contrôle à 50 nM | 35,70 | 3,30 |
| Séquence de contrôle à 0,1 nM | 33,65 | 3,90 |

Résultat : seul le contrôle positif (long dsRNA de 1 kb) induit une expression significative du gène hIFIT-1 détecté à une valeur de cycle seuil d'environ 25. Les séquences testées (SEQ. ID. n°1, 2, 4, 13 et 46) n'induisent l'expression du gène hIFIT-1 que pour des valeurs de cycle seuil d'environ 32-36 soit une différence de 7-11 cycles correspondant à une quantité de messagers 10²-10³ fois plus faible .

Ces essais permettent de conclure que les dsRNA selon l'invention n'induisent pas de réponse type interféron.

## Revendications

1. Composition comprenant au moins un oligonucléotide d'ARN double brin de séquence choisis parmi la SEQ. ID. n°1, 2, 4, 5, 7, 8, 13, 14, 16, 23, 25, 29, 32, 35, 37, 40, 42, 43, 46 ou 47 associé à une particule cationique d'une taille inférieure ou égale à 1 µm, de potentiel zeta compris entre 10 et 80 mV.

2. Composition selon la revendication 1, **caractérisée en ce que** l'oligonucléotide est modifié par l'addition en 2' d'un groupement -O-Methyl.

3. Composition selon la revendication 1 ou 2, **caractérisée en ce que** l'oligonucléotide d'ARN double brin est présent à une concentration inférieure ou égale à 100 µM.

4. Composition selon l'une quelconque des revendications 1 à 3, **caractérisée en ce que** la particule cationique est choisie parmi les micelles de tensioactifs, les micelles de polymères blocs, les liposomes de tensioactifs non ioniques et cationiques, les niosomes, les oléosomes, les particules de nanoémulsions, les nanocapsules, les particules organiques ou les particules inorganiques.

5. Composition selon l'une quelconque des revendications 1 à 4, **caractérisée en ce que** la particule cationique a une taille inférieure ou égale à 500 nm.

6. Composition selon l'une quelconque des revendications 1 à 5, **caractérisée en ce que** la particule cationique a une taille inférieure ou égale à 300 nm.

7. Composition selon l'une quelconque des revendications 1 à 6, **caractérisée en ce que** la particule cationique est un micelle de tensioactifs amphiphiles non ioniques et de tensioactifs cationiques.

8. Composition selon l'une quelconque des revendications 1 à 6, **caractérisée en ce que** le micelle de polymère bloc est choisi parmi un micelle de polymère bloc amphiphile cationique, un micelle de polymère bloc amphiphile non ionique et de polymère bloc amphiphile cationique et un micelle de polymère bloc amphiphile non ionique et de tensioactif cationique.

9. Oligonucléotide d'ARN double brin de séquence choisie parmi la SEQ. ID. n°2, 4, 5, 7, 8, 13, 14, 16, 23, 25, 29, 32, 35, 37, 40, 42, 43, 46 ou 47, destiné à être utilisé pour inhiber l'expression de la tyrosinase.

10. Oligonucléotide d'ARN double brin de séquence choisie parmi la SEQ. ID. n°1, 2, 4, 5, 7, 8, 13, 14, 16, 23, 25, 29, 32, 35, 37, 40, 42, 43, 46 ou 47, destiné à être utilisé pour inhiber l'expression de la tyrosinase, **caractérisée en ce que** ledit oligonucléotide est associé à une particule cationique d'une taille inférieure ou égale à 1 µm, de potentiel zeta compris entre 10 et 80 mV.

11. Oligonucléotide d'ARN selon la revendication 9 ou 10, destiné à être utilisé comme agent blanchissant de la peau et/ou comme agent anti-brunissement.

12. Oligonucléotide d'ARN selon l'une quelconque des revendications 9 à 11, destiné à être utilisé pour éviter la formation et/ou éliminer les taches pigmentaires, les taches de sénescence et/ou pour éclaircir et/ou blanchir et/ou uniformiser la couleur d'une peau brunie.

13. Utilisation d'au moins un oligonucléotide d'ARN double brin de séquence choisie parmi la SEQ. ID. n°2, 4, 5, 7, 8, 13, 14, 16, 23, 25, 29, 32, 35, 37, 40, 42, 43, 46 ou 47, pour la préparation d'une composition destinée à dépigmenter la peau.

14. Utilisation d'au moins un oligonucléotide d'ARN double brin de séquence choisie parmi la SEQ. ID. n°1, 2, 4, 5, 7, 8, 13, 14, 16, 23, 25, 29, 32, 35, 37, 40, 42, 43, 46 ou 47, pour la préparation d'une composition destinée à dépigmenter la peau, **caractérisée en ce que** ledit oligonucléotide est associé à une particule cationique d'une taille inférieure ou égale à 1 µm, de potentiel zeta compris entre 10 et 80 mV.

15. Utilisation selon la revendication 14, **caractérisée en ce que** la composition est destinée à traiter les mélasmas idiopathiques, les hyperpigmentations associées à la grossesse ou à une contraception oestro-progestative, les hyperpigmentations accidentelles, les hyperpigmentations dues à des leucodermies, le vitiligo.

16. Procédé cosmétique pour blanchir et/ou éclaircir le teint et/ou uniformiser la couleur d'une peau brunie comprenant l'application topique d'au moins un oligonucléotide d'ARN double brin de séquence choisis parmi la SEQ. ID. n°2, 4, 5, 7, 8, 13, 14, 16, 23, 25, 29, 32, 35, 37, 40, 42, 43, 46 ou 47.

17. Procédé cosmétique pour blanchir et/ou éclaircir le teint et/ou uniformiser la couleur d'une peau brunie comprenant l'application topique d'au moins un oligonucléotide d'ARN double brin de séquence choisis parmi la SEQ. ID. n°1, 2, 4, 5, 7, 8, 13, 14, 16, 23, 25, 29, 32, 35, 37, 40, 42, 43, 46 ou 47, **caractérisé en ce que** ledit oligonicléotide est associé à une particule cationique d'une taille inférieure ou égale à 1 µm, de potentiel zeta compris entre 10 et 80 mV.

18. Procédé selon la revendication 16 ou 17, **caractérisé en ce que** ledit oligonucléotide est formulé dans une composition topique à une concentration inférieure ou égale à 100 µM.

19. Procédé selon l'une quelconque des revendications 16 à 18, **caractérisé en ce que** l'application topique est précédée d'un prétraitement de la peau choisi parmi un stripping, un peeling chimique, une dermabrasion mécanique, l'application d'un ou plusieurs solvants ayant un effet délipidant, le nettoyage de la peau par un produit moussant détergent.

20. Procédé selon l'une quelconque des revendications 16 à 19, **caractérisé en ce que** l'application topique est suivie par un post traitement occlusif.

## Patentansprüche

1. Zusammensetzung, die mindestens ein doppelsträngiges RNA-Oligonukleotid mit einer aus SEQ ID Nr. 1, 2, 4, 5, 7, 8, 13, 14, 16, 23, 25, 29, 32, 35, 37, 40, 42, 43, 46 oder 47 ausgewählten Sequenz, assoziiert mit einem kationischen Partikel einer Größe von weniger als oder gleich 1 µm und einem Zeta-Potenzial zwischen 10 und 80 mV, umfasst.

2. Zusammensetzung nach Anspruch 1, **dadurch gekennzeichnet, dass** das Oligonukleotid durch Addition einer -O-Methylgruppe an der Position 2' modifiziert ist.

3. Zusammensetzung nach Anspruch 1 oder 2, **dadurch gekennzeichnet, dass** das doppelsträngige RNA-Oligonukleotid in einer Konzentration von weniger als oder gleich 100 µM vorliegt.

4. Zusammensetzung nach einem der Ansprüche 1 bis 3, **dadurch gekennzeichnet, dass** das kationische Partikel aus Tensidmizellen, Blockpolymermizellen, Liposomen aus nichtionischen und kationischen Tensiden, Niosomen, Oleosomen, Nanoemulsionspartikeln, Nanokapseln, organischen Partikeln oder anorganischen Partikeln ausgewählt ist.

5. Zusammensetzung nach einem der Ansprüche 1 bis 4, **dadurch gekennzeichnet, dass** das kationische Partikel eine Größe von weniger als oder gleich 500 nm besitzt.

6. Zusammensetzung nach einem der Ansprüche 1 bis 5, **dadurch gekennzeichnet, dass** das kationische Partikel eine Größe von weniger als oder gleich 300 nm besitzt.

7. Zusammensetzung nach einem der Ansprüche 1 bis 6, **dadurch gekennzeichnet, dass** das kationische Partikel eine Mizelle aus amphiphilen nichtionischen Tensiden und aus kationischen Tensiden ist.

8. Zusammensetzung nach einem der Ansprüche 1 bis 6, **dadurch gekennzeichnet, dass** die Blockpolymermizelle aus einer Mizelle eines amphiphilen kationischen Blockpolymers, einer Mizelle eines amphiphilen nichtionischen Blockpolymers und eines amphiphilen kationischen Blockpolymers und einer Mizelle eines amphiphilen nichtionischen Blockpolymers und eines kationischen Tensids ausgewählt ist.

9. Doppelsträngiges RNA-Oligonukleotid mit einer aus SEQ ID Nr. 2, 4, 5, 7, 8, 13, 14, 16, 23, 25, 29, 32, 35, 37, 40, 42, 43, 46 oder 47 ausgewählten Sequenz für die Verwendung zur Hemmung der Expression von Tyrosinase.

10. Doppelsträngiges RNA-Oligonukleotid mit einer aus SEQ ID Nr. 1, 2, 4, 5, 7, 8, 13, 14, 16, 23, 25, 29, 32, 35, 37, 40, 42, 43, 46 oder 47 ausgewählten Sequenz für die Verwendung zur Hemmung der Expression von Tyrosinase, **dadurch gekennzeichnet, dass** das Oligonukleotid mit einem kationischen Partikel einer Größe von weniger als oder gleich 1 µm und einem Zeta-Potenzial zwischen 10 und 80 mV assoziiert ist.

11. RNA-Oligonukleotid nach Anspruch 9 oder 10 für die Verwendung als Bleichungsmittel der Haut und/oder als Antibräunungsmittel.

12. RNA-Oligonukleotid nach einem der Ansprüche 9 bis 11 für die Verwendung zur Vermeidung der Bildung und/oder zur Beseitigung von Pigmentflecken, Altersflecken und/oder zum Aufhellen und/oder Bleichen und/oder Vereinheitlichen der Farbe von gebräunter Haut.

13. Verwendung mindestens eines doppelsträngigen RNA-Oligonukleotids mit einer aus SEQ ID Nr. 2, 4, 5, 7, 8, 13, 14, 16, 23, 25, 29, 32, 35, 37, 40, 42, 43, 46 oder 47 ausgewählten Sequenz zur Herstellung einer Zusammensetzung für die Depigmentierung der Haut.

14. Verwendung mindestens eines doppelsträngigen RNA-Oligonukleotids mit einer aus SEQ ID Nr. 1, 2, 4, 5, 7, 8, 13, 14, 16, 23, 25, 29, 32, 35, 37, 40, 42, 43, 46 oder 47 ausgewählten Sequenz zur Herstellung einer Zusammensetzung für die Depigmentierung der Haut, **dadurch gekennzeichnet, dass** das Oligonukleotid mit einem kationischen Partikel einer Größe von weniger als oder gleich 1 µm und einem Zeta-Potenzial zwischen 10 und 80 mV assoziiert ist.

15. Verwendung nach Anspruch 14, **dadurch gekennzeichnet, dass** die Zusammensetzung zur Behandlung von idiopathischen Melasmen, Hyperpigmentierungen in Zusammenhang mit einer Schwangerschaft oder einer Östrogen-Gestagen-Empfängnisverhütung, zufälligen Hyperpigmentierungen, durch Leukodermien hervorgerufenen Hyperpigmentierungen und/oder Vitiligo bestimmt ist.

16. Kosmetisches Verfahren zum Bleichen und/oder Aufhellen des Teint und/oder zur Vereinheitlichung der Farbe von gebräunter Haut, welches das topische Auftragen mindestens eines doppelsträngigen RNA-Oligonukleotids mit einer aus SEQ ID Nr. 2, 4, 5, 7, 8, 13, 14, 16, 23, 25, 29, 32, 35, 37, 40, 42, 43, 46 oder 47 ausgewählten Sequenz umfasst.

17. Kosmetisches Verfahren zum Bleichen und/oder Aufhellen des Teint und/oder zur Vereinheitlichung der Farbe von gebräunter Haut, welches das topische Auftragen mindestens eines doppelsträngigen RNA-Oligonukleotids mit einer aus SEQ ID Nr. 1, 2, 4, 5, 7, 8, 13, 14, 16, 23, 25, 29, 32, 35, 37, 40, 42, 43, 46 oder 47 ausgewählten Sequenz umfasst, **dadurch gekennzeichnet, dass** das Oligonukleotid mit einem kationischen Partikel einer Größe von weniger als oder gleich 1 µm und einem Zeta-Potenzial zwischen 10 und 80 mV assoziiert ist.

18. Verfahren nach Anspruch 16 oder 17, **dadurch gekennzeichnet, dass** das Oligonukleotid in einer topischen Zusammensetzung in einer Konzentration von weniger als oder gleich 100 µM formuliert ist.

19. Verfahren nach einem der Ansprüche 16 bis 18, **dadurch gekennzeichnet, dass** dem topischen Auftragen eine Vorbehandlung der Haut vorausgeht, die aus Abziehen, chemischem Peeling, mechanischer Dermabrasion, Aufbringen von einem oder mehreren Lösungsmitteln mit entfettender Wirkung, Reinigung der Haut mit einem schäumenden Detergenzprodukt ausgewählt ist.

20. Verfahren nach einem der Ansprüche 16 bis 19, **dadurch gekennzeichnet, dass** auf das topische Auftragen eine okklusive Behandlung folgt.

## Claims

1. Composition comprising at least one double-stranded RNA oligonucleotide of sequence chosen from SEQ ID NOS. 1, 2, 4, 5, 7, 8, 13, 14, 16, 23, 25, 29, 32, 35, 37, 40, 42, 43, 46 or 47 associated with a cationic particle less than or equal to 1 µm in size, with a zeta potential of between 10 and 80 mV.

2. Composition according to Claim 1, **characterized in that** the oligonucleotide is modified through the addition of an -O-methyl group in the 2'-position.

3. Composition according to Claim 1 or 2, **characterized in that** the double-stranded RNA oligonucleotide is present at a concentration of less than or equal to 100 µM.

4. Composition according to any one of Claims 1 to 3, **characterized in that** the cationic particle is chosen from surfactant micelles, block polymer micelles, liposomes of nonionic and cationic surfactants, niosomes, oleosomes, particles of nanoemulsions, nanocapsules, organic particles or inorganic particles.

5. Composition according to any one of Claims 1 to 4, **characterized in that** the cationic particle is less than or equal to 500 nm in size.

6. Composition according to any one of Claims 1 to 5, **characterized in that** the cationic particle is less than or equal to 300 nm in size.

7. Composition according to any one of Claims 1 to 6, **characterized in that** the cationic particle is a micelle of nonionic amphiphilic surfactants and of cationic surfactants.

8. Composition according to any one of Claims 1 to 6, **characterized in that** the block polymer micelle is chosen from a micelle of a cationic amphiphilic block polymer, a micelle of a nonionic amphiphilic block polymer and of a cationic amphiliphic block polymer and a micelle of a nonionic amphiphilic block polymer and of a cationic surfactant.

9. Double-stranded RNA oligonucleotide of sequence chosen from SEQ ID NOS. 2, 4, 5, 7, 8, 13, 14, 16, 23, 25, 29, 32, 35, 37, 40, 42, 43, 46 or 47, intended to be used for inhibiting tyrosinase expression.

10. Double-stranded RNA oligonucleotide of sequence chosen from SEQ ID NOS. 1, 2, 4, 5, 7, 8, 13, 14, 16, 23, 25, 29, 32, 35, 37, 40, 42, 43, 46 or 47, intended to be used for inhibiting tyrosinase expression, **characterized in that** said oligonucleotide is associated with a cationic particle less than or equal to 1 µm in size, with a zeta potential of between 10 and 80 mV.

11. RNA oligonucleotide according to Claim 9 or 10, intended to be used as a bleaching agent for the skin and/or as an anti-browning agent.

12. RNA oligonucleotide according to any one of Claims 9 to 11, intended to be used for preventing the formation of and/or eliminating pigmentation marks, and/or senescence marks, and/or for lightening and/or bleaching browned skin and/or making the colour of browned skin uniform.

13. Use of at least one double-stranded RNA oligonucleotide of sequence chosen from SEQ ID NOS. 2, 4, 5, 7, 8, 13, 14, 16, 23, 25, 29, 32, 35, 37, 40, 42, 43, 46 or 47, for the preparation of a composition for use in depigmenting the skin.

14. Use of at least one double-stranded RNA oligonucleotide of sequence chosen from SEQ ID NOS. 1, 2, 4, 5, 7, 8, 13, 14, 16, 23, 25, 29, 32, 35, 37, 40, 42, 43, 46 or 47, for the preparation of a composition for use in depigmenting the skin, **characterized in that** said oligonucleotide is associated with a cationic particle less than or equal to 1 µm in size, with a zeta potential of between 10 and 80 mV.

15. Use according to Claim 14, **characterized in that** the composition is for use in treating idiopathic melasmas, hyperpigmentations associated with pregnancy or with oestro-progestin contraception, accidental hyperpigmentations, hyperpigmentations due to leukoderma, and/or vitiligo.

16. Cosmetic method for bleaching and/or lightening the complexion and/or making the colour of a browned skin uniform, comprising the topical application of at least one double-stranded RNA oligonucleotide of sequence chosen from SEQ ID NOS. 2, 4, 5, 7, 8, 13, 14, 16, 23, 25, 29, 32, 35, 37, 40, 42, 43, 46 or 47.

17. Cosmetic method for bleaching and/or lightening the complexion and/or making the colour of a browned skin uniform, comprising the topical application of at least one double-stranded RNA oligonucleotide of sequence chosen from SEQ ID NOS. 1, 2, 4, 5, 7, 8, 13, 14, 16, 23, 25, 29, 32, 35, 37, 40, 42, 43, 46 or 47, **characterized in that** said oligonucleotide is associated with a cationic particle less than or equal to 1 µm in size, with a zeta potential of between 10 and 80 mV.

18. Method according to Claim 16 or 17, **characterized in that** said oligonucleotide is formulated in a topical composition at a concentration of less than or equal to 100 µM.

19. Method according to any one of Claims 16 to 18, **characterized in that** the topical application is preceded by a pretreatment of the skin chosen from a stripping, a chemical peel, a mechanical dermabrasion, the application of one or more solvents having a defatting effect, and cleansing the skin with a detergent foaming product.

20. Method according to any one of Claims 16 to 19, **characterized in that** the topical application is followed by an occlusive post-treatment.
